# EUROPEAN PATENT SPECIFICATION

(11) **EP 3 092 941 B1**
(45) Date of publication and mention of the grant of the patent: **22.01.2020**
(21) Application number: 14870438.0
(22) Date of filing: 04.12.2014
(51) Int. Cl.: A61B 3/10, A61B 3/15, A61B 3/16, A61B 3/103, A61B 3/107

(54) **OPERATING AN OPHTHALMIC DEVICE**
BETRIEB EINER AUGENMEDIZINISCHEN VORRICHTUNG
OPÉRATION D'UN DISPOSITIF OPHTALMIQUE

(30) Priority: 13.12.2013 JP 2013258737
(43) Date of publication of application: 16.11.2016
(73) Proprietor: Kabushiki Kaisha Topcon, Tokyo 174-8580 (JP)
(72) Inventor: NAITO, Tomoko, Tokyo 174-8580 (JP)
(74) Representative: Louis Pöhlau Lohrentz
(86) International application number: PCT/JP2014/082159
(87) International publication number: WO 2015/087783

(56) References cited:
- EP-A2- 2 005 880
- JP-A- H0 994 226
- JP-A- 2002 253 506
- JP-A- 2004 024 471
- JP-A- 2007 282 671
- JP-A- 2010 148 589
- JP-A- 2012 249 809

## Description

### TECHNICAL FIELD

This invention is related to a method for operating a complex ophthalmologic apparatus including a first measurement unit set at a first set operating distance and a second measurement unit set at a second set operating distance.

### BACKGROUND ART

A complex ophthalmologic apparatus including a first measurement unit set at a first set operating distance and a second measurement unit set at a second set operating distance has been proposed (refer to Patent Document 1, for example). The ophthalmologic apparatus includes an intraocular pressure measurement unit as the second measurement unit and an ocular characteristic measurement unit as the first measurement unit. The intraocular pressure measurement unit is set at the second set operating distance having a very small value and the ocular characteristic measurement unit is set at the first set operating distance having a relatively large value. In the ophthalmologic apparatus, the first measurement unit is automatically moved to appropriately measure both subject eyes of a subject, and the second measurement unit is also automatically moved to appropriately measure both subject eyes of the subject. In the ophthalmologic apparatus, the subject eyes are thereby simply and smoothly measured by the first and second measurement units. Likewise, an ophthalmic apparatus capable of performing efficient measurement of a plurality of eye characteristics of an examinee's eye, which comprises a first measurement unit comprising a first measurement system for performing measurement of a first characteristic of the eye and a second measurement unit comprising a second measurement system for performing measurement of a second characteristic of the eye is known from Patent Document EP 2005880 A2.

### CITATION LIST

### Patent Literature

Patent Literature 1: JP2010-148589A

### SUMMARY

### Technical Problem

In the conventional ophthalmologic apparatus of Patent Literature 1, it is necessary to automatically move the first and second measurement units from a position suitable to one subject eye to a position suitable to the other subject eye. This movement distance, namely, the distance between both subject eyes differs depending on a subject. It is therefore considered to move the first and second measurement units with an average value of distances between both subject eyes of many subjects. It is however difficult to appropriately move the first and second measurement units for all subjects.

For this reason, it is considered to detect, as a target for movement, a subject eye (for example, pupil) with the image of the subject eye obtained by an observation optical system in each of the first and second measurement units. However, as the second measurement unit is set at the second set operating distance having a very small value, the second measurement unit is required to move back in the movement from the position suitable to one subject eye to the position suitable to the other subject eye to move away from the subject for avoiding the interference with a nose or the like of the subject. In the second measurement unit, the observation optical system thus detects the subject eye (for example, pupil) at a distance significantly larger than the second set operating distance, so that the observation optical system may not appropriately detect the subject eye (for example, pupil).

The present invention has been made in view of the above circumferences, and an object of the present invention is to provide a method for operating an ophthalmologic apparatus in which the second measurement unit set at the second set operating distance having a value smaller than that of the first set operating distance is automatically and appropriately moved from the position suitable to one subject eye to the position suitable to the other subject eye.

### Solution to Problem

To solve the above problem, an ophthalmologic apparatus includes a first measurement unit set at a first set operating distance to measure a subject eye of a subject, a second measurement unit set at a second set operating distance shorter than the first set operating distance to measure the subject eye, a driver that moves the first measurement unit and the second measurement unit relative to the subject eye, and a controller that controls the first measurement unit, the second measurement unit, and the driver, wherein the controller obtains a distance between both eyes of right and left subject eyes when the first measurement unit is moved to measure the right and left subject eyes of the subject, and after one subject eye is measured with the second measurement unit, the controller moves the second measurement unit to the other subject eye at the obtained distance between both eyes.

### Advantageous Effects

According to the method of operating an ophthalmologic apparatus of the present invention, that is defined in claim 1, the second measurement unit set at the second set operating distance having a value smaller than that of the first set operating distance is automatically and appropriately moved from the position suitable to one subject eye to the position suitable to the other subject eye E.

### BRIEF DESCRIPTION OF DRAWINGS

[FIG. 1] FIG. 1 is a view schematically showing a configuration of an ophthalmologic apparatus 10 of an embodiment according to the present invention.
[FIG. 2] FIG. 2 is a view for explaining an optical configuration of an intraocular pressure measurement unit 20 of the ophthalmologic apparatus 10.
[FIG. 3] FIG. 3 is a view, as seen from a direction different from FIG. 2, for explaining the optical configuration of the intraocular pressure measurement unit 20 of the ophthalmologic apparatus 10.
[FIG. 4] FIG. 4 is a view for explaining an optical configuration of an ocular characteristic measurement unit 40 of the ophthalmologic apparatus 10.
[FIG. 5] FIG. 5 is a view for explaining a switching operation of a measurement mode by moving an apparatus main body 13.
[FIG.6] FIG. 6 is a view for explaining a predetermined height HL and a predetermined forward position FL with a positional relationship between the apparatus main body 13 and a forehead pad 16.
[FIG. 7] FIG. 7 is a view for explaining a switching operation of an ocular characteristic measurement unit 40 of the ophthalmologic apparatus 10 from a position suitable to a left subject eye EL to a position suitable to a right subject eye ER.
[FIG. 8] FIG. 8 is a view for explaining movement of both subject eyes E in an image (data) obtained by an observation optical system 42 when the apparatus main body 13 (ocular characteristic measurement unit 40) is moved from the subject eye EL to the subject eye ER.
[FIG. 9] FIG. 9 is a flowchart showing a right and left eyes switching operation process (right and left eyes switching operation method) that is executed by a controller in the embodiment.
[FIG. 10] FIG. 10 is a view for explaining a switching operation of the intraocular pressure measurement unit 20 of the ophthalmologic apparatus 10 from the position suitable to the right subject eye ER to the position suitable to the left subject eye EL.
[FIG. 11] FIG. 11 is a view showing an unfocused airflow blowing nozzle 21b in an image (data) obtained by an anterior eye observation optical system 21.

### DESCRIPTION OF EMBODIMENT

Hereinafter, an embodiment of an ophthalmologic apparatus according to the present invention, that is defined in claim 1, will be described with reference to the drawings.

### Embodiment

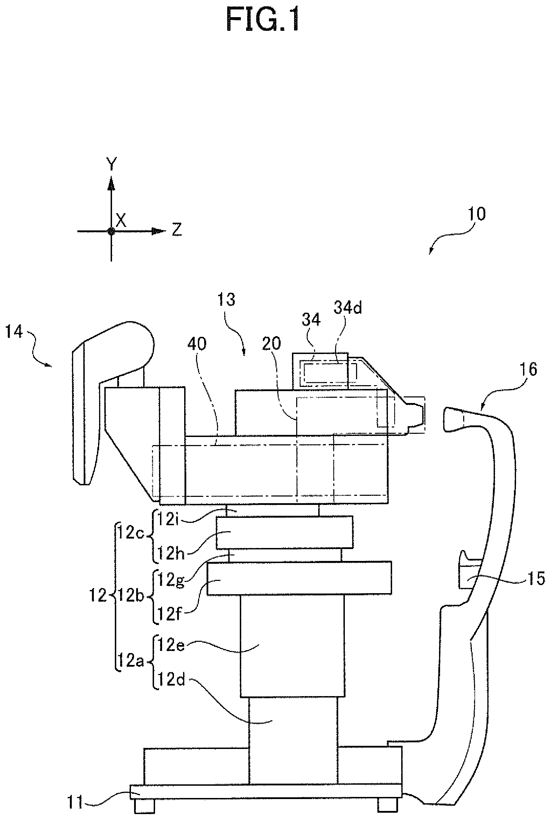

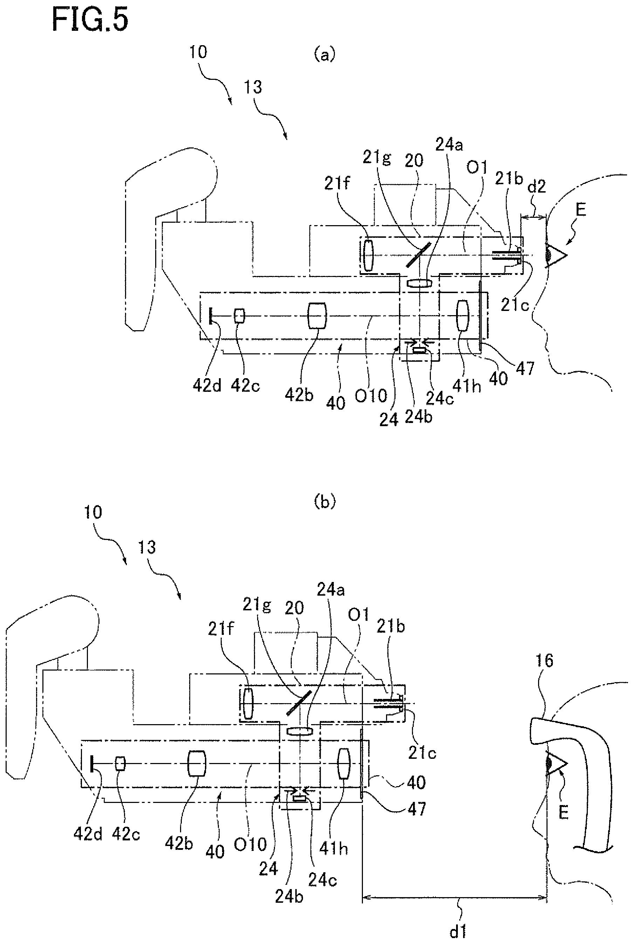

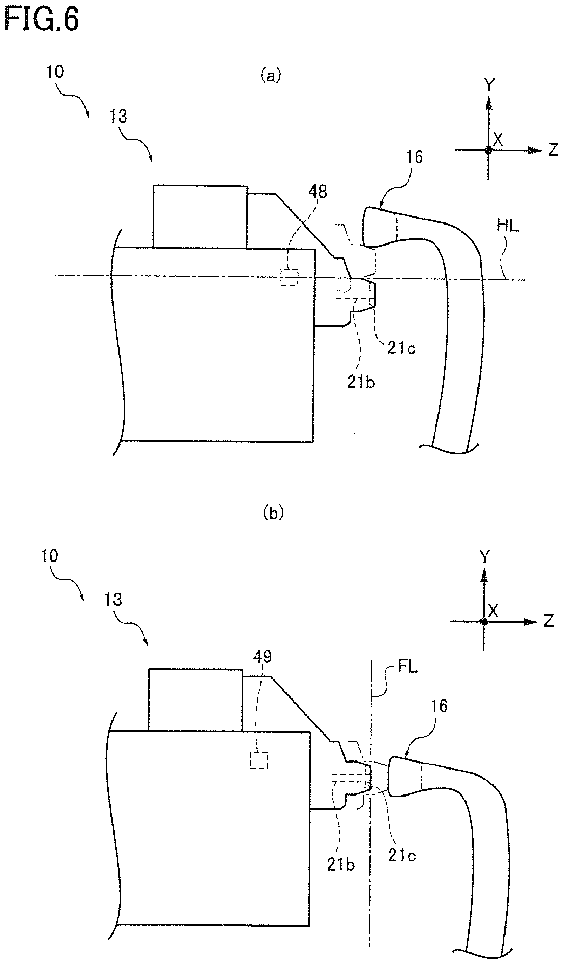

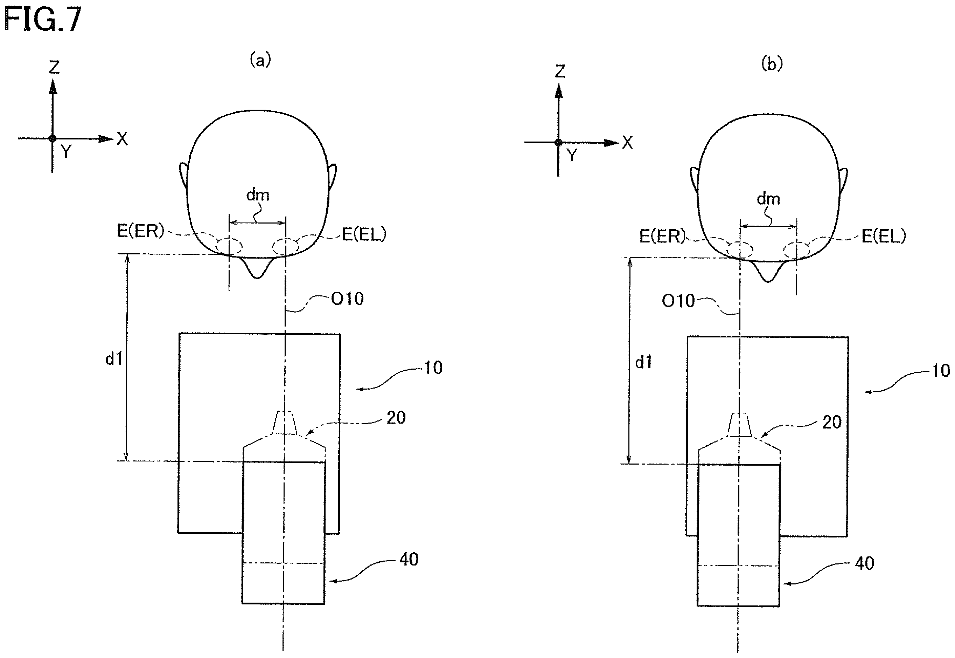

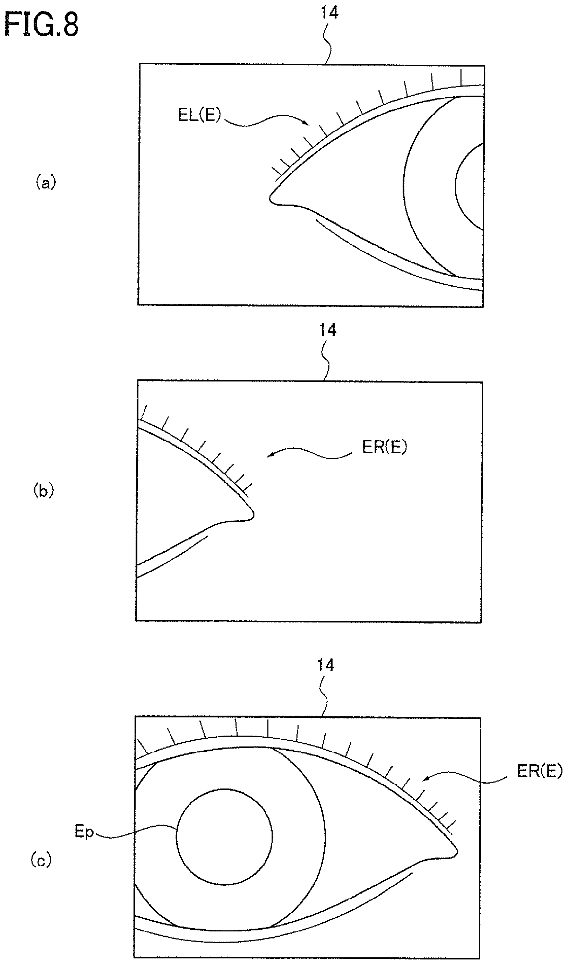

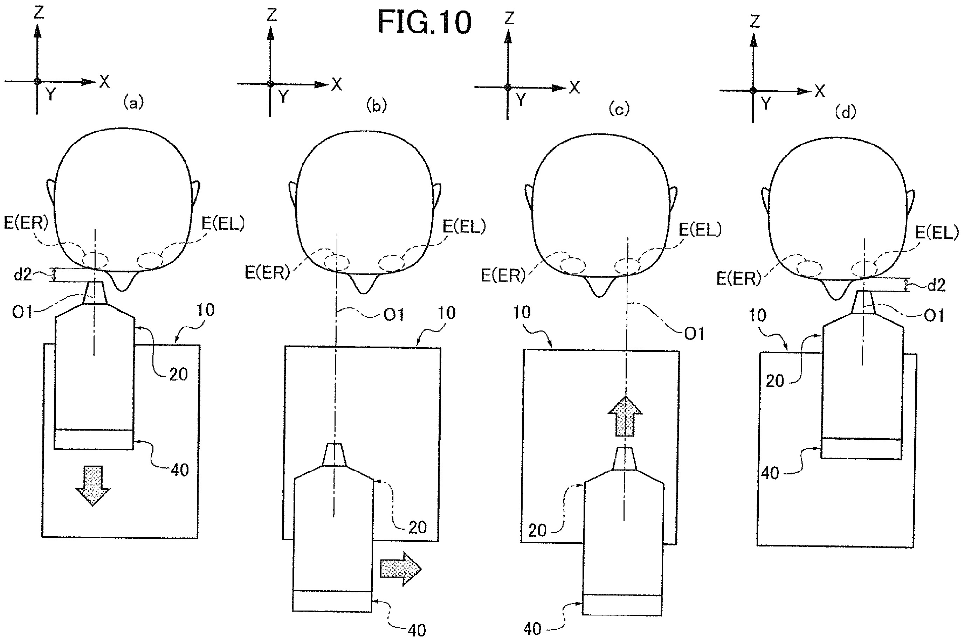

An ophthalmologic apparatus 10 according to the present invention will be described with reference to FIGs. 1 to 11. FIG. 5(a) shows an intraocular pressure measurement mode and FIG. 5(b) shows an ocular characteristic measurement mode. FIG. 6(a) shows a predetermined height HL and FIG. 6(b) shows a predetermined forward position FL. FIG. 7(a) shows a main optical axis 010 of an ocular characteristic measurement unit 40 that is aligned with a left subject eye EL and FIG. 7(b) shows the main optical axis 010 of the ocular characteristic measurement unit 40 that is aligned with a right subject eye ER. FIG. 8(a) shows an image from which the subject eye EL comes away, FIG. 8(b) shows an image in which the subject eye ER comes after FIG. 8(a), and FIG. 8(c) shows an image in which a pupil Ep of the subject eye ER comes after FIG. 8(b). FIG. 10(a) shows an optical axis O1 of an intraocular pressure measurement unit 20 aligned to the right subject eye ER, FIG. 10(b) shows the intraocular pressure measurement unit 20 moved back from FIG. 10(a), FIG. 10(c) shows the intraocular pressure measurement unit 20 moved in the X-axis direction from FIG. 10(b) and the optical axis O1 of the intraocular pressure measurement unit 20 aligned to the left subject eye EL, and FIG 10(d) shows the intraocular pressure measurement unit 20 moved to the subject eye EL from FIG. 10(c).

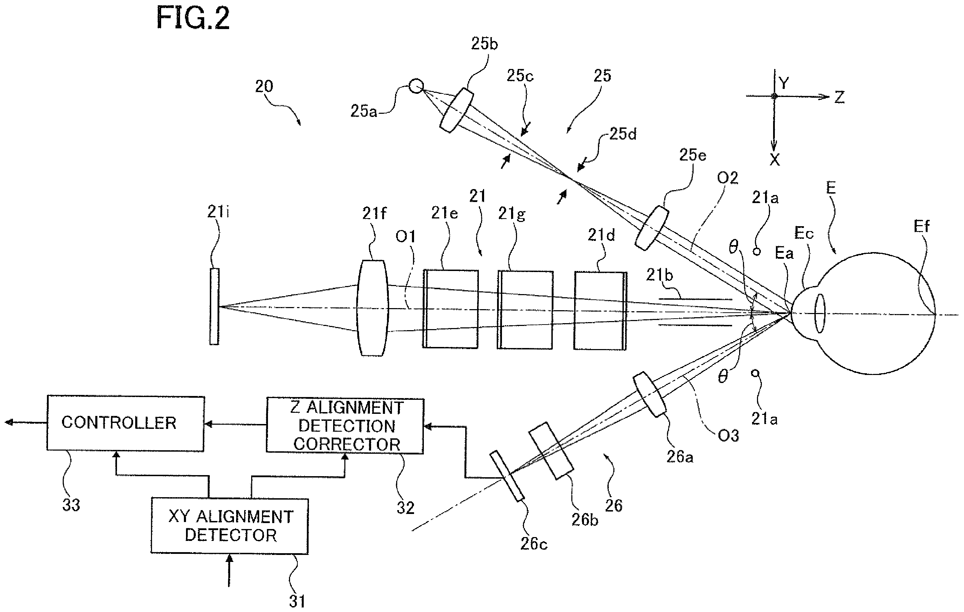

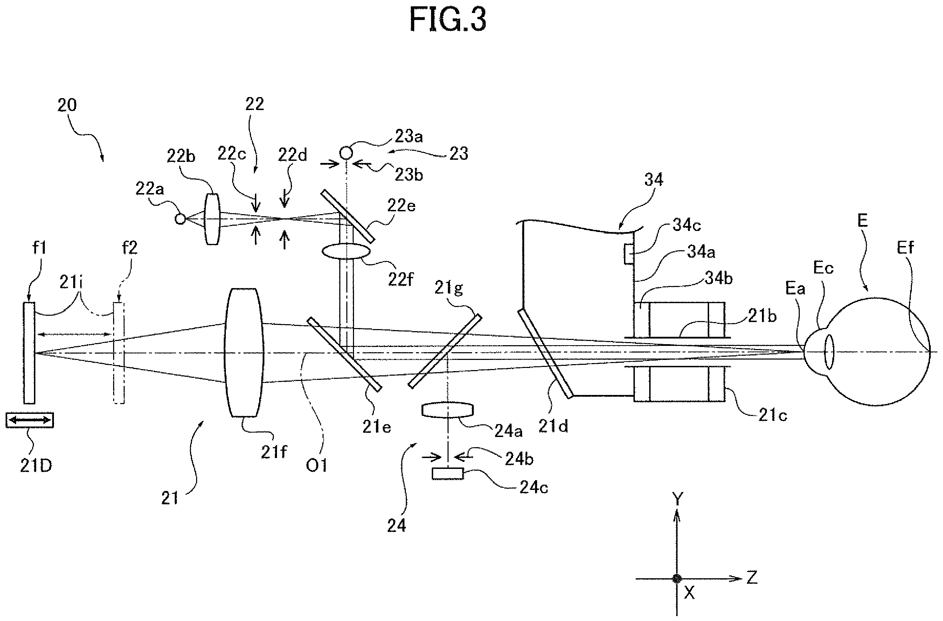

The ophthalmologic apparatus 10 is a complex ophthalmologic apparatus including the intraocular pressure measurement unit 20 that measures an intraocular pressure of a subject eye E (refer to FIG. 2) as one example of a second measurement unit, and the ocular characteristic measurement unit 40 that measures other optical characteristics (ocular characteristic) of the subject eye E as one example of a first measurement unit, as shown in FIG. 1. The subject eye E shows a fundus Ef (retina), a cornea (anterior eye) Ec, and a cornea apex Ea in FIGs. 2 and 3. In the ophthalmologic apparatus 10, an apparatus main body 13 is provided in a base 11 through a driver 12. The apparatus main body 13 is provided with inside thereof the intraocular pressure measurement unit 20 and the ocular characteristic measurement unit 40, and outside thereof a display 14, a jaw receiver 15, and a forehead pad 16.

The display 14 is a liquid crystal display, and displays an image of the anterior eye, an examination result, and the like of the subject eye E under control of a controller 33 described later (refer to FIG. 2). In the present embodiment, the display 14 has a touch panel function that enables an operation for measuring the subject eye E with the intraocular pressure measurement unit 20 or the ocular characteristic measurement unit 40 and an operation for manually moving the apparatus main body 13. The display 14 displays a switching icon of each measurement mode described later with a touch panel function to enable a switching operation of each measurement mode through the touch of the switching icon. In addition, a measurement switch may be provided in the ophthalmologic apparatus 10, and the operation for measuring the subject eye E may be performed through the operation of the measurement switch. A control lever or a movement operation switch may be also provided in the ophthalmologic apparatus 10, and the operation for moving the apparatus main body 13 may be performed through the control lever or the movement operation switch.

The jaw receiver 15 and the forehead pad 16 fix a face of a subject (patient), namely, the position of the subject eye E relative to the apparatus main body 13 in the measurement, and are fixed to the base 11. The jaw receiver 15 is a receiver on which a jaw of the subject is placed, and the forehead pad 16 is a pad on which a forehead of the subject is put. In the ophthalmologic apparatus 10, the display 14, the jaw receiver 15, and the forehead pad 16 are provided across the apparatus main body 13. In regular use, the display 14 faces an examiner while the jaw receiver 15 and the forehead pad 16 face the subject. The display 14 is rotatably supported by the apparatus main body 13 to change a direction of a display surface, for example to face the display surface to the subject or to face the display surface to the side (X-axis direction). The apparatus main body 13 is movable relative to the base 11 by the driver 12 and also relative to the subject eye E (face of subject) fixed by the jaw receiver 15 and the forehead pad 16.

The driver 12 drives the apparatus main body 13 relative to the base 11 in the up and down direction (Y-axis direction), the back and forth direction (Z-axis direction (right and left direction as viewed FIG. 1 from front)), and the right and left direction orthogonal to these directions (X-axis direction (direction orthogonal to FIG. 1)). In this embodiment, the upper side in the up and down direction is the positive side in the Y-axis direction, the subject side (right side as viewed FIG. 1 from front) in the back and forth direction is the positive side in the Z-axis direction, and the front side as viewed FIG. 1 from the front in the right and left direction is the positive side in the X-axis direction (refer to arrows in FIG. 1). In this embodiment, the driver 12 includes a Y-axis driver 12a, a Z-axis driver 12b, and an X-axis driver 12c.

The Y-axis driver 12a is provided in the base 11 to move (displace) the apparatus main body 13 in the Y-axis direction (up and down direction) relative to the base 11 through the Z-axis driver 12b and the X-axis driver 12c. That is the Y-axis driver 12a of the driver 12 is used to move the apparatus main body 13 in the Y-axis direction (up and down direction). The Y-axis driver 12a includes a support column 12d and a Y-axis movement frame 12e. The support column 12d is fixed to the base 11 to extend from the base 11 in the Y-axis direction. The Y-axis movement frame 12e has a shape capable of surrounding the support column 12d, and is attached to the support column 12d to be relatively movable in the Y-axis direction through a not-shown Y-axis guide member. The Y-axis movement frame 12e has a movable range relative to the support column 12d (base 11) in the Y-axis direction, that enables the intraocular pressure measurement unit 20 to be moved in the lowest position (negative side) in an intraocular pressure measurement mode described later (refer to FIG. 5(a)), and the ocular characteristic measurement unit 40 to be moved in the highest position (positive side) in an ocular characteristic measurement mode described later (refer to FIG. 5(b)).

A not-shown elastic member is provided between the Y-axis movement frame 12e and the support column 12d. The Y-axis movement frame 12e is pressed upward by the elastic member. In this embodiment, the elastic member is a tension spring that is made of a spiral wire rod, and that shrinks most with no load and has an elastic force against a behavior that separates one end and the other end. Namely, the Y-axis movement frame 12e is hung from the support column 12d by the elastic member. The Y-axis movement frame 12e is supported by the support column 12d (base 11) through the elastic member while its movement direction relative to the support column 12d (base 11) is defined in the Y-axis direction by the Y-axis guide member. The elastic member is not limited to this embodiment, and may be a compression spring as long as it presses the Y-axis movement frame 12e relative to the support column 12d (base 11) in the Y-axis direction to support the Y-axis movement frame 12e. The compression spring is made of a spiral wire rod, extends most with no load, and has an elastic force against a behavior that brings one end and the other end close to each other. When the compression spring is used, the Y-axis movement frame 12e is supported by the support column 12d or the base 11 from underneath through the compression spring.

The Y-axis driver 12a is provided with a driving force transmission mechanism that applies a movement force for moving the Y-axis movement frame 12e relative to the support column 12d in the Y-axis direction. In the Y-axis driver 12a, the Y-axis movement frame 12e is moved from the elastic member balanced position to the positive side in the Y-axis direction by applying the upward movement force from the driving force transmission mechanism to the Y-axis movement frame 12e, and the Y-axis movement frame 12e is moved from the elastic force balanced position to the negative side in the Y-axis direction by applying the downward movement force from the driving force transmission mechanism to the Y-axis movement frame 12e.

The Z-axis driver 12b is provided in the Y-axis movement frame 12e (Y-axis driver 12a), and moves (displaces) the apparatus main body 13 in the Z-axis direction (back and forth direction) relative to the Y-axis driver 12a, namely, the base 11. That is the Z-axis driver 12b of the driver 12 is used to move the apparatus main body 13 in the Z-axis direction (back and forth direction). The Z-axis driver 12b includes a Z-axis support base 12f and a Z-axis movement base 12g. The Z-axis support base 12f is fixed to the Y-axis movement frame 12e, and is moved in the Y-axis direction together with the Y-axis movement frame 12e. The Z-axis support base 12f supports the Z-axis movement base 12g to be relatively movable in the Z-axis direction through a not-shown Z-axis guide member. The Z-axis driver 12b is provided with a driving force transmission mechanism that applies a movement force for moving the Z-axis movement base 12g relative to the Z-axis support base 12f in the Z-axis direction. In the Z-axis driver 12b, the Z-axis movement base 12g is appropriately moved in the Z-axis direction by applying the movement force in the Z-axis direction from the driving force transmission mechanism to the Z-axis movement base 12g.

The X-axis driver 12c is provided in the Z-axis movement base 12g (Z-axis driver 12b), and moves (displaces) the apparatus main body 13 relative to the Z-axis driver 12b, namely, the base 11 in the X-axis direction (right and left direction). That is the X-axis driver 12c of the driver 12 is used to move the apparatus main body 13 in the X-axis direction (right and left direction). The X-axis driver 12c includes an X-axis support base 12h and an X-axis movement base 12i. The X-axis support base 12h is fixed to the Z-axis movement base 12g, and is moved in the Z-axis direction together with the Z-axis movement base 12g. The X-axis support base 12h supports the X-axis movement base 12i to be relatively movable in the X-axis direction through a not shown X-axis guide member. The X-axis driver 12c is provided with a driving force transmission mechanism that applies a movement force for moving the X-axis movement base 12i relative to the X-axis support base 12h in the X-axis direction. In the X-axis driver 12c, the X-axis movement base 12i is appropriately moved in the X-axis direction by applying the movement force in the Z-axis direction from the driving force transmission mechanism to the X-axis movement base 12i. The apparatus main body 13 is fixed to the X-axis movement base 12i through a not-shown attachment substrate on which the intraocular pressure measurement unit 20 and the ocular characteristic measurement unit 40 provided inside the apparatus main body 13 are mounted.

The driver 12 appropriately moves the apparatus main body 13 in the up and down direction (Y-axis direction), the back and forth direction (Z-axis direction), and the right and left direction (X-axis direction) relative to the base 11 by appropriately driving the Y-axis driver 12a, the Z-axis driver 12b, and the X-axis driver 12c. In the driver 12, as is not shown in the figures, the Y-axis driver 12a, the Z-axis driver 12b, and the X-axis driver 12c are connected to the controller 33 (refer to FIG. 2), and each of the drivers is driven under the control of the controller 33. The controller 33 constitutes an electric control system in the ophthalmologic apparatus 10, and integrally controls the respective parts of the ophthalmologic apparatus 10 by a program stored in a built-in memory.

As is not shown in FIG. 1 for simplifying the description, the ophthalmologic apparatus 10 is provided with a cover member that forms an entire external shape, and is covered by the cover member from the base 11 to the apparatus main body 13 through the driver 12. In FIG. 1, in the driver 12, the Y-axis driver 12a, the Z-axis driver 12b, and the X-axis driver 12c are stacked in the Y-axis direction. However, they are not completely separated in the Y-axis direction, and are configured such that the respective parts are overlapped to each other in the direction orthogonal to the Y-axis direction. In the driver 12, namely, the ophthalmologic apparatus 10, the apparatus main body 13 can be appropriately moved relative to the base 11 while preventing an increase in height (size as seen in the Y-axis direction).

The apparatus main body 13 is provided with inside thereof the intraocular pressure measurement unit 20 and the ocular characteristic measurement unit 40. The ocular characteristic measurement unit 40 constitutes the first measurement unit set at the first set operating distance d1 (refer to FIG. 5(b)) and the intraocular pressure measurement unit 20 constitutes the second measurement unit set at a second set operating distance d2 (refer to FIG. 5(a)) shorter than the first set operating distance. Each operating distance is a distance that enables the measurement of the subject eye E by each measurement unit, and is a distance (interval) from the leading end of each measurement unit to the subject eye E. The intraocular pressure measurement unit 20 is used for measuring the intraocular pressure of the subject eye E. The ocular characteristic measurement unit 40 is used for measuring the optical characteristic (ocular characteristic) of the subject eye E. In this embodiment, the ocular characteristic measurement unit 40 is used for measuring the shape of the cornea Ec of the subject eye E and the refractive power (spherical power, cylindrical power, cylindrical axis angle, and the like) of the subject eye E.

The later-described optical axis O1 as the main optical axis of the intraocular pressure measurement unit 20 is provided above (positive side in Y-axis direction) the later-described main optical axis 010 of the ocular characteristic measurement unit 40 (refer to FIG. 5) in the apparatus main body 13 of the ophthalmologic apparatus 10 of the present embodiment. The intraocular pressure measurement unit 20 is therefore basically provided above the ocular characteristic measurement unit 40 in the apparatus main body 13 of the ophthalmologic apparatus 10. In this case, the intraocular pressure measurement unit 20 and the ocular characteristic measurement unit 40 are not separated, and they have an integrated structure in which a part of the intraocular pressure measurement unit 20 and a part of the ocular characteristic measurement unit 40 are integrated by crossing a part of one optical system to the other optical system in the intraocular pressure measurement unit 20 and the ocular characteristic measurement unit 40.

Next, the optical configuration of the intraocular pressure measurement unit 20 will be described with reference to FIGs. 2 and 3. The intraocular pressure measurement unit 20 is a non-contact intraocular pressure meter. As shown in FIGs. 2 and 3, the intraocular pressure measurement unit 20 includes an anterior eye observation optical system 21 as one example of a second observation optical system, an XY alignment index projection optical system 22, a fixation target projection optical system 23, an applanation detection optical system 24, a Z alignment index projection optical system 25, and a Z alignment detection optical system 26.

The anterior eye observation optical system 21 is provided for observing the anterior eye of the subject eye E and the XY alignment (alignment in direction along X-Y plane). The anterior eye observation optical system 21 is provided with an anterior eye illumination light source 21a (refer to FIG. 2), and also is provided, on the optical axis O1, with an airflow blowing nozzle 21b, an anterior eye window glass 21c (refer to FIG. 3), a chamber window glass 21d, a half mirror 21e, a half mirror 21g, an objective lens 21f, and a CCD camera 21i. A plurality of the anterior eye illumination light sources 21a (only two in FIG. 2) is provided around the anterior eye window glass 21c (refer to FIG. 2) to directly illuminate the anterior eye. The airflow blowing nozzle 21b is a nozzle for blowing airflow to the subject eye E (anterior eye), and is provided in an air compression room 34a (refer to FIG. 3) of an airflow blowing mechanism 34 described later. The CCD camera 21i generates an image signal based on an image (for example, anterior eye image) formed on a light-receiving surface, and outputs the generated image signal to the controller 33 (refer to FIG. 2). The image obtained by the CCD camera 21i is appropriately displayed on the display 14 (refer to FIG. 1), and is appropriately output to a not-shown external device under the control of the controller 33.

As shown in FIG. 3, the CCD camera 21i is movable along the optical axis O1 by a focusing mechanism 21D. The focusing mechanism 21D appropriately moves the CCD camera 21i to be focused on the anterior eye (cornea Ec) of the subject eye E under the control of the controller 33 (refer to FIG. 2). The focusing mechanism 21D shears a driving source with a fixation target movement mechanism 41D of a fixation target projection optical system 41 described later. Namely, the driving source drives the focusing mechanism 21D in the later-described intraocular pressure measurement mode (refer to FIG. 5(a)), and drives the fixation target movement mechanism 41D in the later-described ocular characteristic measurement mode (refer to FIG. 5(b)). The controller 33 changes the position of the CCD camera 21i on the optical axis O1 according to the position of the intraocular pressure measurement unit 20, namely, the apparatus main body 13 to focus the CCD camera 21i on the anterior eye (cornea Ec) of the subject eye E through the focusing mechanism 21D.

The controller 33 displaces the CCD camera 21i through the focusing mechanism 21D in two positions of a first focal position f1 and a second focal position f2 on the optical axis 01. The movement of the CCD camera 21i on the optical axis O1 through the focusing mechanism 21D may be switched by the two steps of the first focal position f1 and the second focal position f2, or the CCD camera 21i may be continuously moved between the first focal position f1 and the second focal position f2.

The first focal position f1 is a position that allows the intraocular pressure measurement unit 20 (apparatus main body 13) to measure the intraocular pressure of the subject eye E, namely, a position where the CCD camera 21i is focused on the anterior eye (cornea Ec) of the subject eye E when the intraocular pressure measurement unit 20 is set at the second set operating distance d2 (refer to FIG. 5(a)) from the anterior eye (cornea Ec) of the subject eye E. In this embodiment, the position that allows the intraocular pressure measurement unit 20 to measure the intraocular pressure of the subject eye E is set at 11 mm of the distance (interval) from the leading end of the airflow blowing nozzle 21b to the subject eye E, namely, the second set operating distance d2 (refer to FIG. 5(a)).

The second focal position f2 is a position where the CCD camera 21i is focused on the anterior eye (cornea Ec) of the subject eye E when the intraocular pressure measurement unit 20 (apparatus main body 13) sufficiently separates from the subject eye E (subject). When the ocular characteristic measurement mode (measurement mode by ocular characteristic measurement unit 40) is switched to the intraocular pressure measurement mode (measurement mode by intraocular pressure measurement unit 20), the intraocular pressure measurement unit 20 (apparatus main body 13) is at first moved in the Y-axis direction to be a height corresponding to the subject eye E, and is then moved in the Z-axis direction to come close to the subject eye E. Such movement is performed for preventing the airflow blowing nozzle 21b (leading end) that extremely comes close to the subject eye E from contacting with the subject eye E. When the subject eye E as the measurement target is switched between the right and left eyes of the subject in the intraocular pressure measurement mode, the intraocular pressure measurement unit 20 is first moved back (negative side in Z-axis direction) from the position where one intraocular pressure is measured, is moved in the right and left direction (X-axis direction), and is moved in the direction coming close to the other subject eye E for the alignment. Such movement is performed for preventing the leading end of the airflow blowing nozzle 21b of the intraocular pressure measurement unit 20 from contacting with the subject (for example, nose) by mistake.

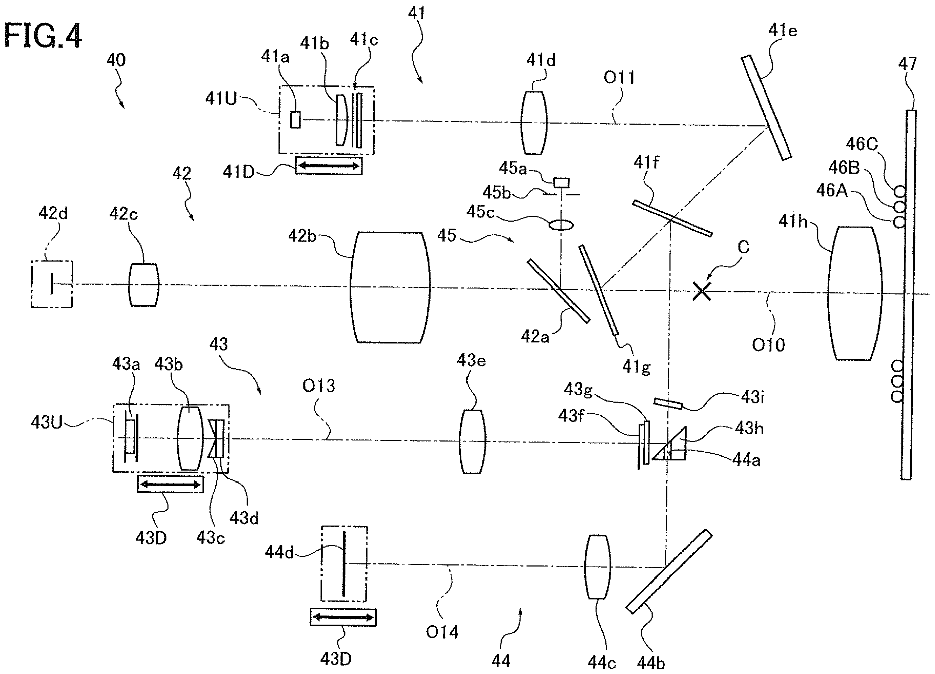

The controller 33 switches the position of the CCD camera 21i between the first focal position f1 and the second focal position f2 according to the position of the apparatus main body 13 (intraocular pressure measurement unit 20) relative to the base 11, namely, the control position in the Z-axis direction (back and forth direction) by the driver 12 (Z-axis driver 12). This is because that the interval between the intraocular pressure measurement unit 20 and the subject eye E is roughly determined based on the position of the apparatus main body 13 (intraocular pressure measurement unit 20) relative to the base 11. In addition, the focusing mechanism 21D shares a driving force with the later-described fixation target movement mechanism 41D (refer to FIG. 4) of the fixation target projection optical system 41. However, such a configuration is not limited to the present embodiment. The focusing mechanism 21D may share the driving source with an index movement mechanism 43D (refer to FIG. 4) of a ring index projection system 43 for measuring refractive power (light-receiving optical system 44) described later.

In the anterior eye observation optical system 21, the anterior eye image of the subject eye E is obtained by the CCD camera 21i while illuminating the subject eye E (anterior eye) by the anterior eye illumination light source 21a (refer to FIG. 2), The anterior eye image (light flux) passes outside the airflow blowing nozzle 21b, transmits through the anterior eye window glass 21c (including later-described glass plate 34b), the chamber window glass 21d, the half mirror 21g, and the half mirror 21e, is focused by the objective lens 21f, and is formed on the CCD camera 21i (light-receiving surface). The CCD camera 21i (anterior eye observation optical system 21) outputs to the controller 33 (refer to FIG. 2) the signal based on the formed anterior eye image. The controller 33 appropriately displays the anterior eye image obtained by the CCD camera 21i (anterior eye observation optical system 21) on the display 14 (refer to FIG. 1). The anterior eye observation optical system 21 therefore operates as the second observation optical system in the intraocular pressure measurement unit 20, namely, the second measurement unit. The anterior eye observation optical system 21 (second observation optical system) may have a magnification higher than that of an observation optical system 42 as a first observation optical system described later in the ocular characteristic measurement unit 40 (first measurement unit). This is because that the intraocular pressure measurement unit 20 (second measurement unit) is required to have alignment accuracy higher than that of the ocular characteristic measurement unit 40 (first measurement unit) in view of the fact that the measurement result of the intraocular pressure of the subject eye E of the intraocular pressure measurement unit 20 (second measurement unit) is easily affected by a decrease in the alignment accuracy.

In the anterior eye observation optical system 21, reflection light of XY alignment index light, which is projected on the subject eye E by the XY alignment index projection optical system 22 and is reflected by the cornea Ec, travels to the CCD camera 21i (light-receiving surface). More specifically, in the anterior eye observation optical system 21, the reflection light flux passes inside the airflow blowing nozzle 21b, and transmits through the chamber window glass 21d, the half mirror 21g, and the half mirror 21e to reach the objective lens 21f. In the anterior eye observation optical system 21, the reflection light flux is focused by the objective lens 21f, and travels to the CCD camera 21i. Then, a bright point image is formed on the CCD camera 21i (light-receiving surface) in the position according to the positional relationship between the cornea Ec and the apparatus main body 13 in the XY direction. The CCD camera 21i (anterior eye observation optical system 21) outputs to the controller 33 (refer to FIG. 2) the signal based on the formed bright point image. The bright point image of the XY alignment index light is formed on the cornea Ex of the subject eye E. The controller 33 therefore obtains the image (data) of the anterior eye (cornea Ec) in which the bright point image is formed, and appropriately displays the image of the anterior eye (cornea Ec) in which the bright point image is formed on the display 14. In addition, an alignment auxiliary mark generated by a not-shown image generator is overlapped with the image to be displayed on the display 14.

The XY alignment index projection optical system 22 projects index light to the cornea Ec of the subject eye E from the front. The index light has a function of adjusting the position of the subject eye E (anterior eye (cornea Ec)) as seen in the direction along the X-Y plane relative to the intraocular pressure measurement unit 20, namely, alignment function in the X and Y directions. The index light also has a function of detecting the deformation (deformation level (applanation)) of the cornea Ec of the subject eye E. The XY alignment index projection optical system 22 includes an XY alignment light source 22a, a condensing lens 22b, an aperture stop 22c, a pinhole plate 22d, a dichroic mirror 22e, and a projection lens 22f, and shares the half mirror 21e with the anterior eye observation optical system 21 and. The XY alignment light source 22a emits infrared light. The projection lens 22f is disposed on the optical axis of the XY alignment index projection optical system 22 to be focused on the pinhole plate 22d. In the XY alignment index projection optical system 22, the infrared light emitted from the XY alignment light source 22a passes through the aperture stop 22c while being condensed by the condensing lens 22b, and travels to the pinhole plate 22d (hole). In the XY alignment index projection optical system 22, the light flux passed thorough the pinhole plate 22d (hole) is reflected by the dichroic mirror 22e, and travels to the projection lens 22f, and the traveled infrared light travels to the half mirror 21e as the parallel light flux. In the XY alignment index projection optical system 22, the parallel light flux is reflected by the half mirror 21e to travel on the optical axis O1 of the anterior eye observation optical system 21. The parallel light flux transmits through the half mirror 21g and the chamber window glass 21d, travels to the inside of the airflow blowing nozzle 21b, and passes through the inside of the airflow blowing nozzle 21b to reach the subject eye E as the XY alignment index light. As is not shown in the figures, the XY alignment index light is reflected by the surface of the cornea Ec to form the bright point image in the intermediate position between the cornea apex Ea of the cornea Ec and the curvature center of the cornea Ec. In addition, the aperture stop 22c is provided in a position conjugate to the cornea apex Ea of the cornea Ec with respect to the projection lens 22f.

The fixation target projection optical system 23 projects (presents) a fixation target to the subject eye E. The fixation target projection optical system 23 includes a fixation target light source 23a and a pinhole plate 23b, and shears the dichroic mirror 22e and the projection lens 22f with the XY alignment index projection optical system 22, and also shears the half mirror 21e with the anterior eye observation optical system 21. The fixation target light source 23a emits visible light. In the fixation target projection optical system 23, the fixation target light emitted from the fixation target light source 23a travels to the pinhole plate 23b (hole), passes through the pinhole plate 23b (hole), transmits through the dichroic mirror 22e, and travels to the projection lens 22f. The fixation target light (light flux) is changed to substantial parallel light by the projection lens 22f, travels to the half mirror 21e, and is reflected by the half mirror 21e to travel on the optical axis O1 of the anterior eye observation optical system 21. The light flux transmits through the half mirror 21g and the chamber window glass 21d, travels to the inside of the airflow blowing nozzle 21b, passes through the inside of the airflow blowing nozzle 21b, and reaches the subject eye E. The subject pays close attention to the fixation target projected on the subject eye E as a fixation target by the fixation target projection optical system 23, and the visual line of the subject is thereby fixed.

As shown in FIG. 3, the XY alignment index projection optical system 22 projects the XY alignment index light to the subject eye E. The applanation detection optical system 24 receives the XY alignment index light reflected by the cornea Ec, and detects the deformation (applanation) of the surface of the cornea Ec. The applanation detection optical system 24 includes a lens 24a, a pinhole plate 24b, a sensor 24c, and a half mirror 21g provided on the optical axis of the anterior eye observation optical system 21. The lens 24a condenses the XY alignment index light reflected by the cornea Ec to the center hole of the pinhole plate 24b when only the surface of the cornea Ec is flat. The pinhole plate 24b is provided to have the center hole in the condensed position by the lens 24a. The sensor 24c is a light-receiving sensor capable of detecting light volume, outputs the signal according the received light volume, and uses a photodiode in this embodiment. This sensor 24c (applanation detection optical system 24) outputs the signal according to the received light volume to the controller 33.

As described above, the reflection light flux of the XY alignment index light reflected by the surface (cornea surface) of the cornea Ec of the subject eye E passes through the inside of the airflow blowing nozzle 21b, transmits through the chamber window glass 21d, and reaches the half mirror 21g. In the applanation detection optical system 24, a part of the light is reflected by the half mirror 21g, travels to the lens 24a, is condensed by the lens 24a, and travels to the pinhole plate 24b. In this case, the surface of the cornea Ec gradually flattens by blowing the airflow to the cornea Ec of the subject eye E from the airflow blowing nozzle 21b with the later-described airflow blowing mechanism 34 (refer to FIG. 1). In this case, in the applanation detection optical system 24, the entire reflection light flux reaches the sensor 24c through the hole of the pinhole plate 24b in a state in which the surface of the cornea Ec flattens, and reaches the sensor 24c while a part of the reflection light flux is blocked by the pinhole plate 24b in other state. The applanation detection optical system 24 detects that the surface of the cornea Ec flattens (applanation) by detecting the maximum light volume received by the sensor 24c. The applanation detection optical system 24 thereby detects the shape (applanation) of the surface of the cornea Ec deformed by the airflow blowing, and the sensor 24c operates as a light receiver that receives the reflection light (reflection light flux) from the cornea Ec for detection.

The Z alignment index projection optical system 25 obliquely projects the alignment index light (alignment index parallel light flux) in the X-axis direction to the cornea Ec of the subject eye E, as shown in FIG. 2. The Z alignment index projection optical system 25 includes, on an optical axis O2, a Z alignment light source 25a, a condensing lens 25b, an aperture stop 25c, a pinhole plate 25d, and a projection lens 25e. The Z alignment light source 25a is a light source that emits infrared light (for example, wavelength of 860 nm). The aperture stop 25c is provided in a position conjugate to the cornea apex Ea of the cornea Ec relative to the projection lens 25e. The projection lens 25e is disposed to have a focal point aligned with the pinhole plate 25d (hole). In the Z alignment index projection optical system 25, the infrared light (light flux) emitted from the Z alignment light source 25a is condensed by the condensing lens 25b, passes through the aperture stop 25c, and travels to the pinhole plate 25d. In the Z alignment index projection optical system 25, the light flux passed through the pinhole plate 25d (hole) travels to the projection lens 25e to be changed to the parallel light flux, and travels to the cornea Ec as the parallel light flux. The infrared light (light flux (Z alignment index light)) is reflected by the surface of the cornea Ec to form a bright point image located inside the subject eye E,

The Z alignment detection optical system 26 receives the Z alignment index light reflected by the cornea Ec from the direction symmetrical to the optical axis O1 of the anterior eye observation optical system 21, and detects the positional relationship between the apparatus main body 13 (intraocular pressure measurement unit 20) and the cornea Ec in the Z-axis direction. The Z alignment detection optical system 26 includes, on an optical axis O3, an imaging lens 26a, a cylindrical lens 26b, and a sensor 26c. The cylindrical lens 26b has a power in the Y-axis direction. The sensor 26c is a light-receiving sensor capable of detecting a light-receiving position in the light-receiving surface, and is configured by a line sensor or a position sensitive detector (PSD). The sensor 26c is connected to a Z alignment detection corrector 32.

In the Z alignment detection optical system 26, the reflection light flux that is the alignment index light projected by the Z alignment index projection optical system 25 and reflected by the surface of the cornea Ec travels to the imaging lens 26a. In the Z alignment detection optical system 26, the reflection light flux is condensed by the imaging lens 26a, travels to the cylindrical lens 26b, and is condensed in the Y-axis direction by the cylindrical lens 26b to form the bright point image on the sensor 26c. This sensor 26c is provided in a position conjugate to the bright point image formed inside the subject eye E by the Z alignment index projection optical system 25 with respect to the imaging lens 26a in the X-Y plan. The sensor 26c is also provided in a position conjugate to the cornea apex Ea with respect to the imaging lens 26a and the cylindrical lens 26b in the Y-Z plane. Namely, the sensor 26c has a conjugate relationship with the cylindrical lens 26b (the magnification is set such that the image of the aperture stop 25c becomes smaller than that of the sensor 26c), and the reflection light flux on the surface of the cornea Ec effectively enters the sensor 26c even when the cornea Ec is shifted in the Y-axis direction. This sensor 26c (Z alignment detection optical system 26) outputs the signal based on the received bright point image to the Z alignment detection corrector 32.

As shown in FIG. 3, the airflow blowing mechanism 34 includes the air compression room 34a provided with an air compression driver 34d (refer to FIG. 1). The air compression driver 34d includes a piston movable inside the air compression room 34a and a driver that moves the piston, and is provided above the intraocular pressure measurement unit 20 (optical system) in the apparatus main body 13 in this embodiment. The air compression driver 34d compresses air in the air compression room 34a by driving under the control of the controller 33 (not shown). The airflow blowing nozzle 21b is attached to the air compression room 34a through a transparent glass plate 34b, and the chamber window glass 21d is provided in the air compression room 34a to face the airflow blowing nozzle 21b. The air compression room 34a prevents the above function in the anterior eye observation optical system 21 from being disturbed. The air compression room 34a is provided with a pressure sensor 34c that detects the pressure of the air compression room 34a. The pressure sensor 34c is connected to the controller 33 (refer to FIG. 2), and outputs the signal according to the detected pressure to the controller 33, In the airflow blowing mechanism 34, the air compression driver 34d compresses the air inside the air compression room 34a to blow the airflow from the airflow blowing nozzle 21b to the cornea Ec of the subject eye E under the control of the controller 33. In the airflow blowing mechanism 34, the pressure when the airflow blows from the airflow blowing nozzle 21b can be obtained by detecting the pressure in the air compression room 34a with the pressure sensor 34c. In the airflow blowing mechanism 34, airflow having a feature in which a change in pressure relative to a time is predefined may be blown instead of providing the pressure sensor 34c.

The intraocular pressure measurement unit 20 includes a driver (driving mechanism) that controls the lighting of the anterior eye illumination light source 21a, the XY alignment light source 22a, the fixation target light source 23a, and the Z alignment light source 25a. The controller 33 (refer to FIG. 2) is connected to the driver. In the intraocular pressure measurement unit 20, the anterior eye illumination light source 21a, the XY alignment light source 22a, the fixation target light source 23a, and the Z alignment light source 25a are appropriately lighted under the control of the controller 33. In the intraocular pressure measurement unit 20, as described above, the CCD camera 21i is appropriately moved on the optical axis O1 through the focusing mechanism 21D, the process of generating the image based on the image signal output from the CCD camera 21i is executed, and the generated image is appropriately displayed on the display 14 under the control of the controller 33.

The schematic operation for measuring the intraocular pressure of the subject eye E with the intraocular pressure measurement unit 20 will be described next. In addition, the following operation in the intraocular pressure measurement unit 20 is executed under the control of the controller 33 (refer to FIG. 2). At first, a power source switch of the ophthalmologic apparatus 10 is powered on, and the operation for the measurement with the intraocular pressure measurement unit 20 is executed to the display 14. Then, after the intraocular pressure measurement unit 20 is set to the intraocular pressure measurement mode (refer to FIG. 5(a)), the anterior eye illumination light source 21a, the XY alignment light source 22a, the fixation target light source 23a, and the Z alignment light source 25a are appropriately lighted. In this case, in the intraocular pressure measurement unit 20, the flashing of the respective light source 21a, 23a, and 25a is repeated at different cycles to distinguish the light.

In the intraocular pressure measurement unit 20, as shown in FIG. 3, by lighting the fixation target light source 23a of the fixation target projection optical system 23, the fixation target is projected to the subject eye E to visually fix the subject eye E, namely, the visual line of the subject. In the intraocular pressure measurement unit 20, the parallel light flux is also projected to the cornea Ec by lighting the XY alignment light source 22a of the XY alignment index projection optical system 22. In the intraocular pressure measurement unit 20, the reflection light flux reflected by the cornea Ec is received by the CCD camera 21i of the anterior eye observation optical system 21 and the sensor 24c of the applanation detection optical system 24. In the intraocular pressure measurement unit 20, as shown in FIG. 2, the parallel light flux for the alignment in the Z-axis direction is projected on the cornea Ec by lighting the Z alignment light source 25a of the Z alignment index projection optical system 25. In the intraocular pressure measurement unit 20, the reflection light flux reflected by the cornea Ec is received by the sensor 26c of the Z alignment detection optical system 26.

In the intraocular pressure measurement unit 20, the anterior eye of the subject eye E is illuminated, and the anterior eye image of the subject eye E is imaged on the CCD camera 21i by lighting the anterior eye illumination light source 21a of the anterior eye observation optical system 21, In the intraocular pressure measurement unit 20, as is not shown in the figures, the anterior eye image of the subject eye E in which the bright point image of the XY alignment index light is formed and the alignment auxiliary mark are displayed on the display 14. The examiner executes the alignment to reflect the bright point image in the screen of the display 14 by moving the apparatus main body 13 right and left and up and down with the operation of an operation part displayed on the display 14 while looking at the display 14. In the intraocular pressure measurement unit 20, the Z alignment detection corrector 32 calculates the positional relationship between the apparatus main body 13 and the cornea Ec in the Z-axis direction based on the calculation result of the XY alignment detector 31 and the light-receiving signal of the sensor 26c of the Z alignment detection optical system 26. In the intraocular pressure measurement unit 20, the automatic alignment (automatic adjustment of alignment) is executed by appropriately moving the apparatus main body 13 relative to the base 11 in the up and down direction (Y-axis direction), the back and forth direction (Z-axis direction), and the right and left direction (X-axis direction) by appropriately driving the driver 12, namely, the Y-axis driver 12a, the Z-axis driver 12b, and the X-axis driver 12c based on the calculation result output from the Z alignment detector 32 and the position in the X and Y directions obtained from the anterior eye observation optical system 21 under the control of the controller 33.

In the intraocular pressure measurement unit 20, at the completion of the automatic alignment, the controller 33 operates the airflow blowing mechanism 34 to blow the airflow toward the cornea Ec of the subject eye E from the airflow blowing nozzle 21b. Then, the surface of the cornea Ec of the subject eye E gradually flattens (applanation). In a process of gradually flattening (applanation) the cornea Ec, when the surface of the cornea Ec flattens (applanation), the maximum light volume is received by the sensor 24c of the applanation detection optical system 24. In this case, in the intraocular pressure measurement unit 20, the controller 33 determines that the surface of the cornea Ec flattens (applanation) based on a change in the light volume received by the sensor 24c. Namely, in the applanation detection optical system 24 (sensor 24c), the applanation of the cornea Ec can be detected. In the intraocular pressure measurement unit 20, the controller 33 obtains the intraocular pressure of the subject eye E based on the output from the pressure sensor 34c (pressure of blown airflow), and displays the calculation result on the display 14. In addition, the controller 33 may obtain the intraocular pressure of the subject eye E (calculate intraocular pressure value) based on a time from the start of the airflow blowing by the airflow blowing nozzle 21b (airflow blowing mechanism 34) to the detection time of the flattening (applanation) of the surface of the cornea Ec.

The optical configuration of the ocular characteristic measurement unit 40 will be described next with reference to FIG. 4. The ocular characteristic measurement unit 40 measures the shape of the cornea Ec of the subject eye E and the refractive power of the subject eye E (spherical power, cylindrical power, cylindrical axis angle, and the like). As shown in FIG. 4, the ocular characteristic measurement unit 40 includes a fixation target projection optical system 41, an observation optical system 42 as one example of the first observation optical system, the ring index projection system 43 for mearing refractive power, the light-receiving optical system 44, and an alignment light projection system 45. The fixation target projection optical system 41 projects a fixation target to the fundus Ef (refer to FIGs. 2 and 3) of the subject eye E for fixing and fogging the subject eye E. The observation optical system 42 observes the anterior eye (cornea Ec) of the subject eye E. The ring index projection system 43 for measuring refractive power projects pattern light flux as the ring index for measuring refractive power to the fundus Ef of the subject eye E to measure the refractive power of the subject eye E. In the light-receiving optical system 44, the ring index image for measuring refractive power reflected by the fundus Ef of the subject eye E is received by an imaging element 44d described later. The ring index projection system 43 for mearing refractive power and the light-receiving optical system 44 constitute a cornea shape and refractive power measurement optical system together with the observation optical system 42 and ring index projection light sources 46A, 46B, and 46C for measuring a cornea shape described later. The alignment light projection system 45 projects the index light to the subject eye E to detect the alignment state in the X and Y directions. The optical system of the ocular characteristic measurement unit 40 is provided with an operation distance detection optical system for detecting an operation distance between the subject eyes E and the apparatus main body 13.

The fixation target projection optical system 41 includes, on an optical axis O11, a fixation target light source 41a, a collimator lens 41b, an index plate 41c, a relay lens 41d, a mirror 41e, a dichroic mirror 41f, a dichroic mirror 41g, and an objective lens 41h. The index plate 41c is provided with a target for fixing and fogging the subject eye E. The fixation target light source 41a, the collimator lens 41b, and the index plate 41c constitute a fixation target unit 41U, and integrally move along the optical axis O11 of the fixation target projection optical system 41 by the fixation target movement mechanism 41D for fixing and fogging the subject eye E. In addition, the dichroic mirror 41g and the objective lens 41h are positioned on the main optical axis O10 in the later-described ocular characteristic measurement unit 40.

In the fixation target projection optical system 41, after the visible light is emitted from the fixation target light source 41a, and the visible light is changed to the parallel light flux by the collimator lens 41b, the visible light transmits through the index plate 41c to be the target light flux. In the fixation target projection optical system 41, the target light flux is reflected by the mirror 41e after passing through the relay lens 41d, passes through the dichroic mirror 41f, and travels to the dichroic mirror 41g. In the fixation target projection optical system 41, the target light flux is reflected by the dichroic mirror 41g to the main optical axis O10 in the ocular characteristic measurement unit 40, and travels to the subject eye E through the objective lens 41h. The subject pays close attention to the target light flux (fixation target) projected on the subject eye E as the fixation target by the fixation target projection optical system 41, and the visual line of the subject is thereby fixed. The fixation target projection optical system 41 fogs the subject eye E by moving the fixation target unit 41U from the fixation target paid close attention by the subject to the unfocused position.

The observation optical system 42 includes a not-shown illumination light source, and, on the main optical axis O10, a half mirror 42a, a relay lens 42b, an imaging lens 42c, and an imaging element 42d, and shares the objective lens 41h and the dichroic mirror 41g with the fixation target projection optical system 41. The imaging element 42d is a secondary solid state imaging element using a CMOS image sensor in this embodiment.

In the observation optical system 42, the anterior eye (cornea Ec) of the subject E is illuminated by the illumination light flux emitted from the illumination light source, and the illumination light flux reflected by the anterior eye is obtained by the objective lens 41h. In the observation optical system 42, the reflected illumination light flux is imaged on the imaging element 42d (light-receiving surface) by the imaging lens 42c through the objective lens 41h, the dichroic mirror 41g, the half mirror 42a, and the relay lens 42b. The imaging element 42d outputs the image signal based on the obtained image to the controller 33 (refer to FIG. 2). The controller 33 displays the image of the anterior eye (cornea Ec) on the display 14 based on the input image signal. In the observation optical system 42, the image of the anterior eye (cornea Ec) can be formed on the imaging element 42d (light-receiving surface), and the image of the anterior eye can be displayed on the display 14. In addition, the illumination light source of the observation optical system 42 is turned off in the measurement of the refractive power after the alignment is completed. The observation optical system 42 therefore operates as the first observation optical system in the ocular characteristic measurement unit 40, namely, the first measurement unit. The observation optical system 42 (first observation optical system) may have a magnification lower than that of the anterior eye observation optical system 21 as the second observation optical system in the intraocular pressure measurement unit 20. This is because that an improvement in the alignment accuracy similar to that in the intraocular pressure measurement unit 20 (second measurement unit) is not required in the ocular characteristic measurement unit 40 (first measurement unit).

The ring index projection system 43 for measuring refractive power includes a refractive power measurement light source 43a, a lens 43b, a conical prism 43c, a ring index plate 43d, a lens 43e, a bandpass filter 43f, a pupil ring 43g, a hole prism 43h, and a rotary prism 43i, and shares the dichroic mirror 41f, the dichroic mirror 41g, and the objective lens 41h with the fixation target projection optical system 41. The refractive power measurement light source 43a and the pupil ring 43g are disposed in the optical conjugate position, and the ring index plate 43d and the fundus Ef of the subject eye E are disposed in the optical conjugate position. The refractive power measurement light source 43a, the lens 43b, the conical prism 43c, and the ring index plate 43d constitute an index unit 43U. The index unit 43U integrally moves along an optical axis 013 of the ring index projection system 43 for measuring refractive power by an index movement mechanism 43D.

In the ring index projection system 43 for mearing refractive power, the light flux emitted from the refractive power measurement light source 43a is changed to the parallel light flux by the lens 43b, and travels to the ring index plate 43d via the conical prism 43c. The light flux transmits through a ring pattern portion formed in the ring index plate 43d to be the pattern light flux as the ring index for measuring refractive power. In the ring index projection system 43, the pattern light flux travels to the hole prism 43h through the lens 43e, the bandpass filter 43f, and the pupil ring 43g, reflects by the reflection surface of the hole prism 43h, and travels to the dichroic mirror 41f via the rotary prism 43i. In the ring index projection system 43 for measurement refractive power, the pattern light flux is reflected by the dichroic mirror 41g after being reflected by the dichroic mirror 41f, and travels on the main optical axis O10 in the ocular characteristic measurement unit 40, In the ring index projection system 43 for measuring refractive power, the pattern light flux is imaged on the fundus Ef (refer to FIGs. 2 and 3) of the subject eye E by the objective lens 41h.

The ring index projection system 43 for measuring refractive power is provided with the ring index projection light sources 46A, 46B, and 46C for measuring a cornea shape in front of the objective lens 41h. The ring index projection light sources 46A, 46B, and 46C for measuring a cornea shape are arranged on a ring pattern 47 to be concentric to the main optical axis O1 at a predetermined distance from the subject eye E (cornea Ec), and project ring index light for measuring a cornea shape to the subject eye E (cornea Ec). The ring index light for measuring a cornea shape is projected on the cornea Ec of the subject eye E to form the ring index for measuring a cornea shape on the cornea Ec. The ring index (light flux) for measuring a cornea shape is reflected by the cornea Ec of the subject eye E to be imaged on the imaging element 42d by the observation optical system 42. In the observation optical system 42, the image of the ring index for measuring a cornea shape is overlapped with the image of the anterior eye (cornea Ec) and is displayed on the display 14.

The light-receiving optical system 44 includes a hole 44a of the hole prism 43h, a mirror 44b, a lens 44c, and an imaging element 44d, shares the objective lens 41h, the dichroic mirror 41g, and the dichroic mirror 41f with the fixation target projection optical system 41, and shares the rotary prism 43i with the ring index projection system 43 for measuring refractive power. The imaging element 44d is a secondary solid state imaging element using a CCD image sensor in this embodiment. The imaging element 44d moves along the optical axis O1 of the light-receiving optical system 44 in conjugation with the index unit 43U of the ring index projection system 43 for measuring refractive power by the index movement mechanism 43D of the ring index projection system 43 for measuring refractive power.

In the light-receiving optical system 44, the pattern reflection light flux guided to the fundus Ef (refer to FIGs. 2 and 3) by the ring index projection system 43 for measuring refractive power and reflected by the fundus Ef is condensed by the objective lens 41h, is reflected by the dichroic mirror 41f after being reflected by the dichroic mirror 41g, and travels to the rotary prism 43i. In the light-receiving optical system 44, the reflected pattern reflection light flux travels to the hole 44a of the hole prism 43h via the rotary prism 43i, and passes through the hole 44a. In the light-receiving optical system 44, the pattern reflection light flux passed through the hole 44a is reflected by the mirror 44b, and the pattern reflection light flux, namely, the ring index for measuring refractive power is imaged on the imaging element 44d (light-receiving surface) by the lens 44c. The imaging element 44d outputs the image signal based on the obtained image to the controller 33 (refer to FIG, 2). The controller 33 displays the image of the ring index for measuring refractive power on the display 14 (refer to FIG. 1) based on the input image signal. In the light-receiving optical system, the image of the ring index for measuring refractive power can be formed on the imaging element 44d (light-receiving surface), and the image of the ring index for measuring refractive power can be displayed on the display 14.

The alignment light projection system 45 includes an LED 45a, a pinhole 45b, and a lens 45c, and shares the half mirror 42a with the observation optical system 42, and also shares the dichroic mirror 41g and the objective lens 41h with the fixation target projection optical system 41. In the alignment light projection system 45, the light flux from the LED 45a passes through the pinhole 45b (hole) to be the alignment index light flux, is reflected by the half mirror 42a through the lens 45c, and travels on the main optical axis O10 in the ocular characteristic measurement unit 40. In the alignment light projection system 45, the alignment index light flux travels to the objective lens 41h through the dichroic mirror 41g, and is projected to the cornea Ec of the subject eye E through the objective lens 41h. The alignment light projection system 45 has a function of automatically aligning the apparatus main body 13 with the subject eye E by projecting the alignment index light flux to the cornea Ec of the subject eye E. The alignment index light flux projected as the parallel light to the subject eye E is reflected by the cornea Ec of the subject eye E, and the bright point image as the alignment index image is projected on the imaging element 42d by the observation optical system 42. When the bright point image is positioned in the alignment mark formed by a not-shown optical system, the alignment is completed.

The ocular characteristic measurement unit 40 includes a driver (driving mechanism) for controlling the lighting of the fixation target light source 41a, the illumination light source of the observation optical system 42, the refractive power measurement light source 43a, the LED 45a, and the ring index projection light sources 46A, 46B, and 46C for measuring a cornea shape, and the controller 33 (refer to FIG. 2) is connected to the driver. In the ocular characteristic measurement unit 40, the fixation target light source 41a, the illumination light source of the observation optical system 42, the refractive power measurement light source 43a, the LED 45a, and the ring index projection light sources 46A, 46B, and 46C for measuring a cornea shape are therefore appropriately lighted under the control of the controller 33. In the ocular characteristic measurement unit 40, as described above, the fixation target unit 41U is integrally moved along the optical axis O11 through the fixation target movement mechanism 41D, the index unit 43U is integrally moved along the optical axis 013 through the index movement mechanism 43D, and the imaging element 44d is moved along the optical axis 014 under the control of the controller 33. In the ocular characteristic measurement unit 40, as described above, the generation process of the image based on the image signal output from the imaging element 42d and the imaging element 44d is executed, and the generated image is appropriately displayed on the display 14 under the control of the controller 33.

A schematic operation for measuring the shape of the cornea Ex of the subject eye E and the refractive power (spherical diopter power, astigmatic power, astigmatic axis angle, and the like) of the subject eye E with the ocular characteristic measurement unit 40 will be described next. In addition, the following operation in the ocular characteristic measurement unit 40 is executed under the control of the controller 33 (refer to FIG. 2). At first, the power source switch of the ophthalmologic apparatus 10 is turned on, and the operation for the measurement with the ocular characteristic measurement unit 40 is executed to the display 14. After the ocular characteristic measurement unit 40 is set to the ocular characteristic measurement mode (refer to FIG. 5(b)), in the observation optical system 42, the illumination light source is turned on, and the image of the anterior eye (cornea Ec) is displayed on the display 14. The examiner operates the operation part displayed on the display 14 to locate the pupil Ep (refer to FIG. 8) of the subject eye E in the screen of the display 14, so as to schematically align the apparatus main body 13 relative to the subject eye E by moving the apparatus main body 13 up and down and right and left. In the ocular characteristic measurement unit 40 (controller 33), the pupil Ep can be detected from the image of the anterior eye based on the image signal output from the imaging element 42d. In one example, the shape which should be recognized as the pupil Ep is previously recorded in the image of the anterior eye, and the shape which should be recognized is detected based on the contrast in the image. In the ocular characteristic measurement unit 40, when the subject eye E as the measurement target is switched between the right and left eyes, the pupil Ep is detected based on the image while moving the apparatus main body 13 in the right and left direction in accordance with the switching, and the apparatus main body 13 (ocular characteristic measurement unit 40) is moved with the detected pupil Ep as the target position. The above-described rough alignment is thereby automatically achieved.

In the ocular characteristic measurement unit 40, the bright point image as the alignment index image is displayed on the display 14 by the observation optical system 42. After that, in the ocular characteristic measurement unit 40, the alignment detection is started based on the alignment light projection system 45 and the operating distance detection optical system (not shown). Namely, in the ocular characteristic measurement unit 40, the automatic alignment (automatic adjustment of alignment) is executed by appropriately moving the apparatus main body 13 in the up and down direction (Y-axis direction), the back and forth direction (Z-axis direction) and the right and left direction (X-axis direction) relative to the base 11 to position the bright point image as the alignment index image in the alignment mark. In the ocular characteristic measurement unit 40, the automatic alignment of the apparatus main body 13 relative to the apex of the cornea Ec of the subject eye E is thereby completed.

Then, in the ocular characteristic measurement unit 40, the ring index projection light sources 46A, 46B, and 46C for measuring refractive power in the ring index projection system 43 for measuring refractive power are lighted to project the ring indexes for measuring a shape of a cornea to the cornea Ec. In the controller 33, the shape of the cornea Ec is measured from the image of the ring index for measuring a shape of a cornea projected on the cornea Ec based on the image (image signal from imaging element 42d) displayed on the display 14. As the details of the measurement of the shape of the cornea Ec are known, the description thereof will be omitted. The controller 33 measures the spherical power, the cylindrical power, and the cylindrical axis angle as the refractive power by a known method. Note that the configuration of the refractive power measurement unit is the same as that disclosed in JP2002-253506A, but the configuration is not limited thereto. The controller 33 executes the measurement of the refractive power (optical characteristic) as well as the measurement of the cornea shape. In addition, the controller 33 appropriately stores the calculation results in a not-shown memory.

In this embodiment, the intraocular pressure measurement unit 20 is basically provided above the ocular characteristic measurement unit 40, and the intraocular pressure measurement unit 20 and the ocular characteristic measurement unit 40 are fixed to an attachment base in the apparatus main body 13 of the ophthalmologic apparatus 10. More specifically, as the intraocular pressure measurement unit 20 and the ocular characteristic measurement unit 40 are integrally configured in the apparatus main body 13 of the ophthalmologic apparatus 10, it is not necessary to change the positional relationship between the intraocular pressure measurement unit 20 and the ocular characteristic measurement unit 40. The intraocular pressure measurement unit 20 and the ocular characteristic measurement unit 40 (apparatus main body 13) attached to the attachment base are appropriately moved in the up and down direction (Y-axis direction), the back and forth direction (Z-axis direction), and the right and left direction (X-axis direction) by the driver 12, namely, the Y-axis driver 12a, the Z-axis driver 12b, and the X-axis driver 12c. In the ophthalmologic apparatus 10, the intraocular pressure measurement unit 20 and the ocular characteristic measurement unit 40 can be moved to the position corresponding to the subject eye E (face of subject) fixed to the jaw receiver 15 and the forehead pad 16 (refer to FIG. 5) by moving the apparatus main body 13 (attachment base) relative to the base 11 with the driver 12 (Y-axis driver 12a, Z-axis driver 12b, and X-axis driver 12c).

In the ophthalmologic apparatus 10, the subject eye E can be positioned on the extended line of the optical axis O1 of the anterior eye observation optical system 21 of the intraocular pressure measurement unit 20 by moving the apparatus main body 13 (attachment base) relative to the base 11 with the Y-axis driver 12a of the driver 12 in the up and down direction (Y-axis direction), so as to set the intraocular pressure measurement unit 20 to the height position of the subject eye E (refer to FIG. 5(a)). In the ophthalmologic apparatus 10, as shown in FIG. 5(a), the leading end of the airflow blowing nozzle 21b of the anterior eye observation optical system 21 of the intraocular pressure measurement unit 20 can be positioned at the second set operating distance d2 from the subject eye E (cornea apex Ea) by moving the airflow blowing nozzle 21b in the back and forth direction (Z-axis direction) with the Z-axis driver 12b of the driver 12. The second set operating distance d2 is 11 mm in this embodiment. This state is the measurement mode by the intraocular pressure measurement unit 20, namely, the intraocular pressure measurement mode.

In the ophthalmologic apparatus 10, the subject eye E can be positioned on the extended line of the main optical axis O10 of the ocular characteristic measurement unit 40 by moving the apparatus main body 13 (attachment base) relative to the base 11 in the up and down direction (Y-axis direction) with the Y-axis driver 12a of the driver 12, so as to set the ocular characteristic measurement unit 40 in the height position of the subject eye E (refer to FIG. 5(b)). In the ophthalmologic apparatus 10, as shown in FIG. 5(b), the front end of the ocular characteristic measurement unit 40 (ring pattern 47 in this embodiment) can be positioned at the first set operating distance d1 from the subject eye E by moving the ocular characteristic measurement unit 40 in the back and forth direction (Z-axis direction) with the Z-axis driver 12b of the driver 12. The front end of the ocular characteristic measurement unit 40 is a structure closest to the subject in the ocular characteristic measurement unit 40, which is the ring pattern 47 in this embodiment. The first set operating distance d1 is about 80 mm in this embodiment. This state is the measurement mode by the ocular characteristic measurement unit 40, namely, the ocular characteristic measurement mode.

In this embodiment, the front end (airflow nozzle 21b) of the intraocular pressure measurement unit 20 is displaced closer to the positive side (subject side) in the Z-axis direction than the front end (ring pattern 47) of the ocular characteristic measurement unit 40 in the ophthalmologic apparatus 10 for the following reasons. In the ophthalmologic apparatus 10, in the intraocular pressure measurement mode, the ring pattern 47 as the front end of the ocular characteristic measurement unit 40 is positioned in front of the nose or the mouth of the subject, as shown in the FIG. 5(a). In the ophthalmologic apparatus 10, as the intraocular pressure measurement unit 20 and the ocular characteristic measurement unit 40 are integrally configured, the positional relationship between the units is not changed. In the ophthalmologic apparatus 10, when the front end of the intraocular pressure measurement unit 20 and the front end of the ocular characteristic measurement unit 40 are equally positioned in the Z-axis direction (back and forth direction), the ring pattern 47 has contact with the nose or the mouth of the subject or the ring pattern 47 gives uncomfortable feeling to the subject in the intraocular pressure measurement mode. In view of such circumstances, in the ophthalmologic apparatus 10, the front end (airflow blowing nozzle 21b) of the intraocular pressure measurement unit 20 is displaced closer to the positive side in the Z-axis direction than the front end (ring pattern 47) of the ocular characteristic measurement unit 40 (ring pattern 47), so as to acquire a space in front of the nose or the mouth of the subject in the intraocular pressure measurement mode.

Accordingly, in this embodiment, the first set operating distance d1 from the subject eye E (cornea apex Ea) to the front end (ring pattern 47 in this embodiment) of the ocular characteristic measurement unit 40 in the measurement is set to 75 mm or more (in this embodiment, about 80 mm) in the ocular characteristic measurement unit 40 of the ophthalmologic apparatus 10 for the following reasons. The first set operating distance d1 is a distance for enabling the measurement of the subject eye E with the ocular characteristic measurement unit 40, and is a reference position for moving the ocular characteristic measurement unit 40 in the Z-axis direction according to the subject eye E (condition). A movement range for moving the ocular characteristic measurement unit 40 from the first set operating distance d1 to the positive side and the negative side in the Z-axis direction is therefore set, and the movement range in this embodiment is set to ±20 mm. In the ophthalmologic apparatus 10, as described above, the front end (airflow blowing nozzle 21b) of the intraocular pressure measurement unit 20 is displaced closer to the positive side in the Z-axis direction than the front end (ring pattern 47) of the ocular characteristic measurement unit 40. In the ophthalmologic apparatus 10, as shown in FIG. 5(b), in the ocular characteristic measurement mode, the airflow blowing nozzle 21b as the front end of the intraocular pressure measurement unit 20 is thereby positioned to face the forehead pad 16 that fixes the face of the subject together with the jaw receiver 15. In the ophthalmologic apparatus 10, if the first set operating distance d1 in the ocular characteristic measurement unit 40 of the ophthalmologic apparatus 10 is small, the front end (airflow blowing nozzle 21b) of the intraocular pressure measurement unit 20 may interfere with the forehead pad 16 when moving the ocular characteristic measurement unit 40 on the positive side (subject side) in the Z-axis direction with the above movement range. More specifically, in the ophthalmologic apparatus 10, it is necessary to set the first set operating distance d1 to a large value for moving the ocular characteristic measurement unit 40 from the first set operating distance d1 on the positive side in the Z axis direction with the above movement range. For this reason, the first set operating distance d1 is set to 75 mm or more, about 80 mm in this embodiment in the ocular characteristic measurement unit 40 of the ophthalmologic apparatus 10. Such a first set operating distance d1 can be set by adjusting the setting of the optical characteristic of each optical member in the ocular characteristic measurement unit 40. The front end of the intraocular pressure measurement unit 20 is therefore reliably prevented from interfering with the forehead pad 16 in the ocular characteristic measurement mode even though the front end (airflow blowing nozzle 21b) of the intraocular pressure measurement unit 20 is displaced closer to the positive side in the Z-axis direction than the front end of the ocular characteristic measurement unit 40.

In the ophthalmologic apparatus 10, as shown in FIG. 6(a), the apparatus main body 13 is provided with a height detector 48 that detects a predetermined height HL. The height HL is set for preventing the airflow blowing nozzle 21b of the intraocular pressure measurement unit 20 from interfering with the forehead pad 16. In the example shown in FIG. 6(a), the height HL is set to have a sufficient distance between the airflow blowing nozzle 21b and the forehead pad 16, but such a distance can be appropriately set, and is not limited to the present embodiment. When the apparatus main body 13 reaches the height HL, the height detector 48 outputs a corresponding signal to the controller 33 (refer to FIG. 2). Upon the reception of the signal from the height detector 48, the controller 33 stops moving the apparatus main body 13 upward in the intraocular pressure measurement mode or when the intraocular pressure measurement unit 20 is located closer to the positive side (subject side) in the Z-axis direction than a predetermined back and forth position. The predetermined back and forth position is set for preventing the airflow blowing nozzle 21b from interfering with the forehead pad 16 in the Z-axis direction.

When the controller 33 receives the above signal from the height detector 48, the controller 33 stops moving the apparatus main body 13 upward as well as executes the control according to the condition that moves the apparatus main body 13 upward. The control according to the condition is to display on the display 14 that no further upward movement is possible when the controller 33 moves the apparatus main body 13 upward based on the operation on the display 14. The control according to the condition is also to move the apparatus main body 13 upward after moving back the apparatus main body 13 in the back and forth direction (negative side in Z-axis direction) when the controller 33 executes the movement for switching from the intraocular pressure measurement unit 20 to the ocular characteristic measurement unit 40. When the controller 33 receives the signal from the height detector 48 during the automatic alignment in the intraocular pressure measurement unit 20, the subject eye E is redetected. This is because that the apparatus main body 13 that reaches the height HL during the automatic alignment in the intraocular pressure measurement unit 20 means the failure of the detection of the subject eye E as the alignment standard.

When the power source switch of the ophthalmologic apparatus 10 is turned on, the controller 33 determines whether or not the above signal is output from the height detector 48 before moving the apparatus main body 13. When the signal is not output from the height detector 48, the controller 33 appropriately moves the apparatus main body 13. When the signal is output from the height detector 48, the controller 33 executes the control according to the condition without moving the apparatus main body 13 upward. The control according to the condition is to display on the display 14 that no further upward movement is possible when the operation for moving the apparatus main body 13 upward is executed on the display 14. The control according to the condition is also to move the apparatus main body 13 upward after moving back the apparatus main body 13 in the back and forth direction (negative side in Z-axis direction) when the movement for switching from the intraocular pressure measurement unit 20 to the ocular characteristic measurement unit 40 is executed. Accordingly, even when the power source switch of the ophthalmologic apparatus 10 is tuned off while the intraocular pressure measurement unit 20 is switched to the ocular characteristic measurement unit 40 after the height detector 48 outputs the signal, the apparatus main body 13 can be moved while the airflow blowing nozzle 21b is reliably prevented from interfering with the forehead pad 16 when the power source switch is turned on again. In the example illustrated in FIG. 6(a), the height detector 48 is provided in the apparatus main body 13. However, it is not limited to the present embodiment, and the height detector 48 may be provided in the Y-axis driver 12a of the driver 12 that controls the height direction of the apparatus main body 13, namely, the positon in the Y-axis direction, or may be provided in another position.

In the ophthalmologic apparatus 10, as shown in FIG. 6(b), the apparatus main body 13 is provided with a forward position detector 49 that detects a predetermined forward position FL. The forward position FL is set for preventing the airflow blowing nozzle 21b of the intraocular pressure measurement unit 20 from interfering with the forehead pad 16 in the ocular characteristic measurement mode. The forward position FL is set to have a distance between the leading end of the airflow blowing nozzle 21b and the forehead pad 16 in 1 mm. The forward position FL is not limited to this embodiment as long as it is set to have an appropriately distance between the airflow blowing nozzle 21b and the forehead pad 16. When the apparatus main body 13 reaches the forward position FL, the forward position detector 49 outputs a corresponding signal to the controller 33 (refer to FIG. 2). When the controller 33 receives the signal from the forward position detector 49 in the ocular characteristic measurement mode, the controller 33 stops moving the apparatus main body 13 forward (positive side (subject side) in Z-axis direction).

When the controller 33 receives the above signal from the forward position detector 49 in the ocular characteristic measurement mode, the controller 33 stops moving the apparatus main body 13 forward as well as executes the control according to the condition that moves the apparatus main body 13 forward. The control according to the condition is to display on the display 14 that no further forward movement is possible when the apparatus main body 13 is moved forward based on the operation on the display 14. When the power source switch of the ophthalmologic apparatus 10 is turned on, the controller 33 determines whether or not the signal is output from the forward position detector 49 before executing the movement of the apparatus main body 13. The controller 33 appropriately executes the movement of the apparatus main body 13 when the signal is not output from the forward position detector 49. When the signal is output from the forward position detector 49, the controller 33 executes the control according to the condition without moving the apparatus main body 13 forward. The control according to the condition is to display on the display 14 that no further forward movement is possible when the operation for moving the apparatus main body 13 forward is executed on the display 14. In addition, in the example shown in FIG. 6(b), the forward position detector 49 is provided in the apparatus main body 13. However, it is not limited to the present embodiment, and the forward position detector 49 may be provided in the Z-axis driver 12b of the driver 12 that controls the position of the apparatus main body 13 in the back and forth direction, namely, the Z-axis direction, or may be provided in another position.

In the ophthalmologic apparatus 10, the intraocular pressure of the subject eye E is measured by the intraocular pressure measurement unit 20 after the shape of the cornea Ec of the subject eye E and the refractive power (spherical power, cylindrical power, cylindrical axis angle, and the like) of the subject eye E are measured by the ocular characteristic measurement unit 40. In the ophthalmologic apparatus 10, as described above, the controller 33 controls the operation of each part based on the operation on the display 14.

When the power source switch the ophthalmologic apparatus 10 is turned on, an intraocular pressure switching icon as the intraocular pressure measurement mode and an ocular characteristic switching icon as an ocular characteristic measurement mode are displayed on the display 14. A single switching icon or another type icon may be displayed on the display 14 instead of displaying the intraocular pressure switching icon and the ocular characteristic measurement mode. The icon is not limited to the present embodiment as long as it enables the selection and the switching between the intraocular pressure measurement mode and the ocular characteristic measurement mode. The ocular characteristic switching icon is touched (selected) to measure the shape of the cornea Ec of the subject eye E and the refractive power (spherical power, cylindrical power, cylindrical axis angle, and the like) of the subject eye E before measuring the intraocular pressure of the subject eye E.

Then, the ophthalmologic apparatus 10 is set to the ocular characteristic measurement mode (refer to FIG. 5(b)) by appropriately driving the Y-axis driver 12a, the Z-axis driver 12b, and the X-axis driver 12c of the driver 12. Namely, in the ophthalmologic apparatus 10, as shown in FIG. 5(b), the subject eye E is positioned on the extended line of the main optical axis O10 of the ocular characteristic measurement unit 40, and the objective lens 41h of the fixation target projection optical system 41 of the ocular characteristic measurement unit 40 is positioned at the first set operating distance d1 from the subject eye E, so as to set the subject eye E to the ocular characteristic measurement unit 40. The shape of the cornea Ec of the subject eye E and the refractive power (spherical power, cylindrical power, and cylindrical axis angle, and the like) of the subject eye E are then measured by the operation of the ocular characteristic measurement unit 40 in the ophthalmologic apparatus 10. After that, the intraocular pressure switching icon is touched (selected) to measure the intraocular pressure of the subject eye E.

Then, the ophthalmologic apparatus 10 is set to the intraocular pressure measurement mode (refer to FIG. 5(a)) by appropriately driving the Y-axis driver 12a, the Z-axis driver 12b, and the X-axis driver 12c of the driver 12. In the ophthalmologic apparatus 10, at first, the intraocular pressure measurement unit 20 is set to the height corresponding to the subject eye E by moving the apparatus main body 13 on the negative side in the Y-axis direction. In this case, in the ophthalmologic apparatus 10, the position of the CCD camera 21i on the optical axis O1 is set to the second focal position f2 (refer to FIG. 3) through the focusing mechanism 21D. The image of the subject eye E (anterior eye (cornea Ec)) can be thereby appropriately displayed on the display 14. After that, the intraocular pressure measurement unit 20 comes close to the subject eye E by moving the apparatus main body 13 on the positive side in the Z-axis direction. The leading end of the airflow blowing nozzle 21b is then positioned at the second set operating distance d2 from the subject eye E, as shown in FIG. 5(a), so as to set the subject eye to the intraocular pressure measurement unit 20. In this case, the position of the CCD camera 21i on the optical axis O1 is moved to the first focal position f1 (refer to FIG. 3) through the focusing mechanism 21D. The image of the subject eye E (anterior eye (cornea Ec)) can be thereby appropriately displayed on the display 14 in the ophthalmologic apparatus 10. The intraocular pressure of the subject eye E is then measured by the above operation of the intraocular pressure measurement unit 20 in the ophthalmologic apparatus 10.

Accordingly, the shape of the cornea Ec of the subject eye E and the refractive power (spherical power, cylindrical power, and cylindrical axis angle, and the like) of the subject eye E are measured by the ocular characteristic measurement unit 40, and the intraocular pressure of the subject eye E are measured by the intraocular pressure measurement unit 20 in the ophthalmologic apparatus 10.

The characteristic configuration of the ophthalmologic apparatus 10 of the embodiment according to the present invention will be described next with reference to FIGs. 7 to 11. The ocular characteristic measurement unit 40 is set at the first set operating distance d1 in FIG. 7 while the intraocular pressure measurement unit 20 is set at the second set distance d2 in FIG. 10. However, these are shown to simplify the difference in the position relative to the subject based on the difference between the first set operating distance d1 and the second set operating distance d2, and the difference does not always coincide with the difference (movement) in the actual apparatus.

When the ocular characteristic is measured with the ocular characteristic measurement unit 40 (refer to FIG. 4), the image of the anterior eye is obtained by forming the image of the anterior eye (cornea Ec) on the imaging element 42d (light-receiving surface) by the observation optical system 42, and the image (data) is output to the controller 33. The image obtained by the observation optical system 42 (imaging element 42d) can be displayed on the display 14 (refer to FIG. 8) under the control of the controller 33 (refer to FIG. 2) in the ophthalmologic apparatus 10.

In the ophthalmologic apparatus 10, the controller 33 detects the position of the pupil Ep of the subject eye E (cornea Ec) in the input image (data). The shape which should be recognized as the pupil Ep in the image of the anterior eye is previously stored. The pupil Ep is detected by detecting the shape which should be recognized based on the construct in the image. The controller 33 detects the area showing the pupil Ep and the central position in the image of the anterior eye. In addition, the position of the pupil Ep may be detected with another method as long as it detects the position of the pupil Ep in the image (data) obtained by the observation optical system 42 (imaging element 42d). The method is not limited to the present embodiment.

The apparatus main body 13 is schematically aligned with the subject eye E by using the operation for detecting the position of the pupil Ep in the ophthalmologic apparatus 10. One example of the rough alignment will be described with reference to the condition shown in FIG. 7 that measures both subject eyes in order with the ocular characteristic measurement unit 40. In the condition shown in FIG. 7, the left eye (subject eye EL) of the subject is the first measurement target (refer to FIG. 7(a)), and the right eye (subject eye ER) of the subject is the second measurement target (refer to FIG. 7(b)). In this case, the controller 33 moves the apparatus main body 13 (ocular characteristic measurement unit 40) to the subject eye ER (negative side in X-axis direction) after measuring the subject eye EL, and executes the rough alignment to position the apparatus main body 13 according to the subject eye ER. In this case, in the image (data) input to the controller 33, the subject eye EL moves to the right, as shown in FIG. 8(a), and the subject eye ER enters the visual sight (screen of display 14) of the observation optical system 42, as shown in FIG. 8(b). Then, the subject eye ER moves in the central direction of the image (data), as shown in FIG. 8(c), and the pupil Ep of the subject eye ER can be detected. Then, the pupil Ep is detected, and the automatic rough alignment is completed by moving the apparatus main body 13 (ocular characteristic measurement unit 40) with the detected pupil Ep as the target position. In the ocular characteristic measurement unit 40 (controller 33), as described above, the automatic alignment of the apparatus main body 13 relative to the apex of the cornea Ec of the subject eye E can be executed, and the subject eye ER can be measured.

In the ocular characteristic measurement unit 40, as the first set operating distance d1 is set to a large value, as shown in FIG. 7, the apparatus main body 13 (leading end) does not interfere with the subject. The apparatus main body 13 can be therefore moved in the X-axis direction without being moved in the Z-axis direction. In this case, as the observation optical system 42 is provided for the measurement with the ocular characteristic measurement unit 40, the observation optical system 42 is appropriately focused on the subject eye E set at the first set operating distance d1 of the ocular characteristic measurement unit 40. In the ocular characteristic measurement unit 40 (controller 33), the misdetection of the pupil Ep such as the failure of the detection of the pupil is therefore basically prevented.

In the ophthalmologic apparatus 10, the controller 33 stores the position of the apparatus main body 13 in each of the X, Y, and Z directions. The position of the apparatus main body 13 in each of the X, Y, and Z directions is controlled by using the number of pulses of the pulse motor as the driving source in the Y-axis driver 12a, the Z-axis driver 12b, and the X-axis driver 12c of the driver 12. The controller 33 therefore counts the number of pulses from each standard position in each of the X, Y, and Z directions, and stores the position of the apparatus main body 13 in each of the X, Y, and Z directions by storing the count number. These are stored in a memory built in the controller 33 or a memory provided outside the controller 33. The controller 33 stores the position of the apparatus main body 13 in each of the X, Y, and Z directions at the completion of the alignment for measuring the subject eye EL and the position of the apparatus main body 13 in each of the X, Y, and Z directions at the completion of the alignment for measuring the subject eye ER by using the above operation. The controller 33 obtains a distance between both eyes dm (refer to FIG. 7) as a distance between the subject eye EL and the subject eye ER by obtaining the difference in the position of the apparatus main body 13 in each of the X, Y, and Z directions for both of the right and left subject eyes, and stores the obtained distance between both eyes dm. The controller 33 may obtain the distance between both eyes dm by obtaining the difference in the position of the apparatus main body 13 in the X-axis direction for both of the right and left subject eyes. The storing of the position of the apparatus main body 13 in each of the X, Y, and Z directions is not limited to the configuration of the present embodiment, and is appropriately executed according to the configuration of the driver as long as it is executed based on the change in the position of the apparatus main body 13 (ocular characteristic measurement unit 40).

In the ophthalmologic apparatus 10, the controller 33 may obtain the distance between both eyes dm by using the image (data) of the anterior eye (cornea Ec) obtained by the observation optical system 42 (first observation system) of the ocular characteristic measurement unit 40 (first measurement unit). As described later, the distance between both eyes dm is used for switching the right and left subject eyes E in the measurement with the intraocular pressure measurement unit 20 (second measurement unit). The distance between both eyes dm for the switching can be obtained by obtaining the positions of both subject eyes E of the subject. The method of obtaining the distance between both eyes dm with an image (data) includes a method with the center positions of the pupils Ep of both subject eyes E in the image and a method with the cornea apexes Ea (position) of the corneas Ec of both subject eyes E in the image. The controller 33 can detect the center position of the pupil Ep in the image (data) as described above. The center position of the pupil Ep detected by the controller 33 can be used. The cornea apex Ea (position) can be detected by obtaining the position of the bright point image formed by the alignment light projection system 45 in the image (data), namely, the position of the bright point image on the X-Y plane. This is because that the bright point image formed by the alignment light projection system 45 is formed on the axis line passing through the cornea apex Ea and the curvature center of the cornea Ec. The distance between both eyes dm obtained by using the position of the apparatus main body 13 in each of the X, Y, and Z directions at the completion of the alignment is the same as the distance between both eyes dm obtained from the cornea apexes Ea of both subject eyes E by using the position of the apparatus main body 13.

In the ophthalmologic apparatus 10, the controller 33 detects the gap (size and direction) between the main optical axis O10 of the ocular characteristic measurement unit 40 and the subject eye E by using the image (data) of the anterior eye (cornea Ec) obtained by the observation optical system 42 (first observation optical system) of the ocular characteristic measurement unit 40 (first measurement unit). As the center position of the pupil Ep and the cornea apex Ea (position) are obtained as the position of the subject eye E from the image (data), the gap can be obtained by obtaining the gap (size and the direction) between these position and the position of the main optical axis O10 of the present ocular characteristic measurement unit 40. Similarly, the controller 33 detects the gap (size and the direction) between the optical axis O1 of the intraocular pressure measurement unit 20 and the subject eye E by using the image (data) of the anterior eye (cornea Ec) obtained by the anterior eye observation optical system 21 (second observation system) of the intraocular pressure measurement unit 20 (second measurement unit). The controller 33 uses the gap as a correction value when obtaining the position of the apparatus main body 13 in each of the X, Y, and Z directions, and uses the gap as a correction value when moving the apparatus main body 13.

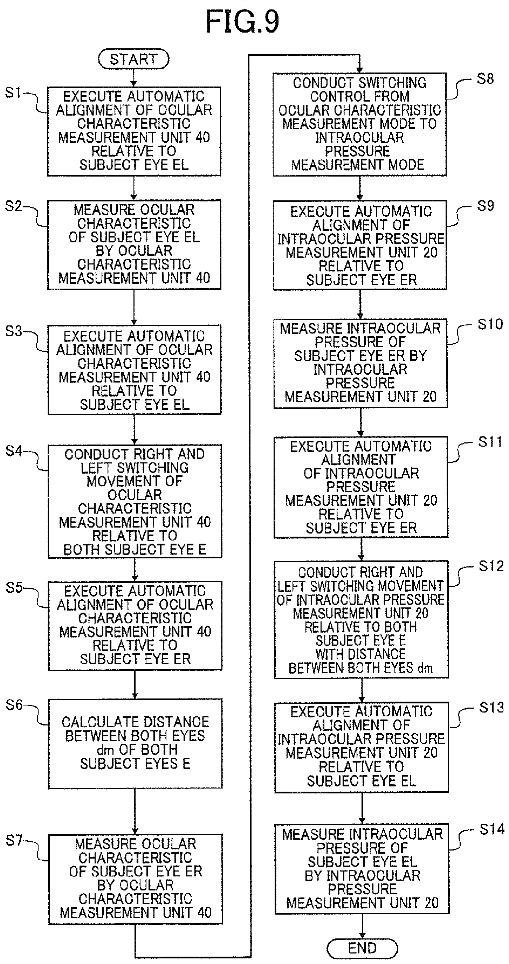

Next, a right and left eyes switching process that executes a right and left eyes switching method in the second measurement unit (intraocular pressure measurement unit 20) as one embodiment will be described with reference to FIG. 9. The right and left eyes switching process is executed by the controller 33, and automatically moves the second measurement unit from the position suitable for one subject eye E to the position suitable for the other subject eye E. FIG. 9 is a flowchart showing the right and left eyes switching process (right and left eyes switching method) which is executed by the controller 33 in this embodiment. The right and left eyes switching process (right and left eyes switching methods) is executed by the controller 33 based on a program stored in the memory built in the controller 33 or in the memory provided outside the controller 33.

The right and left eyes switching process (right and left eyes switching method) basically obtains the distance between both eyes dm of both subject eyes E when measuring the right and left subject eyes E of the subject with the ocular characteristic measurement unit 40 (first measurement unit), and moves the intraocular pressure measurement unit 20 (second measurement unit) in accordance with the obtained distance between both eyes dm for switching the right and left subject eyes E in the measurement with the intraocular pressure measurement unit 20 (second measurement unit). In the right and left eyes switching process (right and left eyes switching method), it is therefore necessary to previously measure the right and left subject eyes E of the subject with the ocular characteristic measurement unit 40 (first measurement unit). In addition, the previous measurement with the ocular characteristic measurement unit 40 is not limited to be executed just before the measurement of the right and left eyes E of the subject with the intraocular pressure measurement unit 20 (second measurement unit), may be measurement that is executed in the past to the subject, and is not limited to the present embodiment. In this case, the distance between both eyes dm in the past measurement to the subject is associated with the subject to be stored.

Hereinafter, each step in the flowchart of FIG. 9 as the right and left eyes switching process (right and left eyes switching method) will be described. The flowchart of FIG. 9 shows an example that measures the subject eye ER (right eye) with the ocular characteristic measurement unit 40 (first measurement unit) after measuring the subject eye EL (left eye), and then measures the subject eye ER (right eye) with the intraocular pressure measurement unit 20 (second measurement unit) after measuring the subject eye EL (left eye). The measurement with the ocular characteristic measurement unit 40 (first measurement unit) may be automatically switched to the measurement with the intraocular pressure measurement unit 20 (second measurement unit) at the completion of the measurement of the right and left eyes with the ocular characteristic measurement unit 40 (first measurement unit), or may be switched by touching the intraocular pressure switching iron as described above. The measurement order of the right and left eyes is not limited to the present embodiment. Moreover, in the measurement with the ocular characteristic measurement unit 40 (first measurement unit) and the intraocular pressure measurement unit 20 (second measurement unit), the right and left eyes E may be switched by an operation for switching. This flowchart (right and left eyes switching process (right and left eyes switching method)) is started in response to an operation for starting the measurement of the subject eye EL with the ocular characteristic measurement unit 40 (first measurement unit).

In Step S1, the automatic alignment of the ocular characteristic measurement unit 40 (first measurement unit) is executed relative to the subject eye EL, and the process proceeds to Step S2. In this step S1, in the ocular characteristic measurement unit 40, the automatic alignment is executed by appropriately moving the apparatus main body 13 relative to the base 11 to position the bright point image as the alignment index image formed in the subject eye EL in the alignment mark based on the alignment light projection system 45 and a not-shown operation distance detection optical system. The subject eye EL is thereby positioned on the main optical axis O1 of the ocular characteristic measurement unit 40, the leading end of the ocular characteristic measurement unit 40 is positioned at the first set operating distance d1 from the subject eye EL, and the ocular characteristic measurement unit 40 is thereby appropriately positioned relative to the subject eye EL.

In Step S2, following the automatic alignment of the ocular characteristic measurement unit 40 (first measurement unit) relative to the subject eye EL in Step S1, the ocular characteristic of the subject eye EL is measured by the ocular characteristic measurement unit 40 (first measurement unit), and then the process proceeds to step S3. In Step S2, as described above, the measurement of the ocular characteristic of the subject eye EL with the ocular characteristic measurement unit 40 is executed. In Step S2 of the present embodiment, the shape of the cornea and the refractive power (optical characteristic) of the subject eye EL are measured by the ocular characteristic measurement unit 40.

In Step S3, following the measurement of the ocular characteristic of the subject eye EL with the ocular characteristic measurement unit 40 (first measurement unit) in Step S2, the automatic alignment of the ocular characteristic measurement unit 40 (first measurement unit) relative to the subject eye EL is executed, and then the process proceeds to Step S4. In step S3, similar to Step S1, the automatic alignment relative to the subject eye EL is executed. The subject eye EL is thereby positioned on the main optical axis O10 of the ocular characteristic measurement unit 40, the leading end of the ocular characteristic measurement unit 40 is positioned at the first set operating distance d1 from the subject eye EL, and the ocular characteristic measurement unit 40 is thereby appropriately positioned relative to the subject eye EL. In step S3, upon the completion of the automatic alignment, the position (coordinate position) of the automatically aligned apparatus main body 13 (ocular characteristic measurement unit 40) in each of the X, Y, and Z-axis directions is stored. In addition, this position to be recoded may be only the position in the X-axis direction. Namely, as Step S3 is a transition step from the measurement of the subject eye EL to the measurement of the subject eye ER, the automatic alignment relative to the subject eye EL is executed for storing the accurate position of the subject eye EL just before the transition. In addition, as described above, when the gap as the correction amount is used by using the image (data) of the anterior eye (cornea Ec) obtained by the observation optical system 42 (first observation system) of the ocular characteristic measurement unit 40 (first measurement unit), or when the distance between both eyes dm is obtained by using that image (data), the ocular characteristic measurement unit 40 moves to the position that detects the position of the bright point image (cornea apex Ea) or the center position of the pupil Ep in the image (data).

In Step S4, following the automatic alignment of the ocular characteristic measurement unit 40 (first measurement unit) relative to the subject eye EL in Step S3, the right and left switching movement of the ocular characteristic measurement unit 40 (first measurement unit) relative to both subject eyes E is conducted, and then the process proceeds to Step S5. In Step S4, as the ocular characteristic of the subject eye ER has not been measured yet although the ocular characteristic of the subject eye EL has been measured, the rough alignment is executed by moving the apparatus main body 13 to the subject eye ER (negative side in X-axis direction) to be appropriately positioned relative to the subject eye ER. More specifically, in Step S4, the apparatus main body 13 is moved to the subject eye ER (negative side in X-axis direction) while executing the detection control of the subject eye ER (pupil Ep) in the image obtained by the observation optical system 42 (imaging element 42d). In Step S4, upon the detection of the pupil Ep of the subject eye ER, the apparatus main body 13 is moved (rough alignment) to position the pupil Ep (center) on the main optical axis O10 of the ocular characteristic measurement unit 40. Namely, in Step S4, the pupil Ep of the subject eye ER is detected, and the apparatus main body 13 (ocular characteristic measurement unit 40) is moved (rough alignment) with the detected pupil Ep (center) as the target position. The right and left switching movement of the subject eye E (from subject eye EL to subject eye ER in this embodiment) is therefore completed.

In Step S5, following the right and left switching movement of the ocular characteristic measurement unit 40 (first measurement unit) relative to both subject eyes E in Step S4, the automatic alignment of the ocular characteristic measurement unit 40 (first measurement unit) relative to the subject eye ER is executed, and then the process proceeds to Step S6. In Step S5, similar to Step S1 and Step S3, the automatic alignment relative to the subject eye ER is executed. The subject eye ER is thereby positioned on the main optical axis 010 of the ocular characteristic measurement unit 40, the leading end of the ocular characteristic measurement unit 40 is positioned at the first set operating distance d1 from the subject eye ER, and the ocular characteristic measurement unit 40 is thereby appropriately positioned relative to the subject eye ER. In Step S5, upon the completion of the automatic alignment, the position of the automatically aligned apparatus main body 13 (ocular characteristic measurement unit 40) in each of the X, Y, and Z-axis directions is stored. In addition, this position to be recoded may be only the position in the X-axis direction.

In Step S6, following the automatic alignment of the ocular characteristic measurement unit 40 (first measurement unit) relative to the subject eye ER in Step S5, the distance between both eyes dm of both subject eyes E (subject eyes EL and ER) is calculated, and then the process proceeds to Step S7. In Step S6, the distance between both eyes dm of both subject eyes E (subject eyes EL and ER) is calculated based on the position of the subject eye EL stored in Step S3 and the position of the subject eye ER stored in Step S5, and the distance between both eyes dm is stored. In Step S6, the distance between both eyes dm is obtained from the cornea apexes Ea of both subject eyes E by using the position of the apparatus main body 13. In addition, as described above, when the distance between both eyes dm is obtained by using the image (data) of the anterior eye (cornea Ec) obtained by the observation optical system 42 (first observation system) of the ocular characteristic measurement unit 40, the center positions of the pupils Ep of both subject eyes E may be used or the cornea apexes Ea (position) of the corneas Ec of both subject eyes E may be used. The intraocular pressure measurement unit 20 (second measurement unit) is to measure the intraocular pressure of the subject eye E with the optical axis O1 being aligned with the cornea apex Ea (position) of the cornea Ec of the subject eye E. In this case, it is therefore preferable to obtain the distance between both eyes dm by using the cornea apexes Ea (position) of the corneas Ec of both subject eyes E.

In Step S7, following the calculation of the distance between both eyes dm of both subject eyes E in Step S6, the ocular characteristic of the subject eye ER is measured by the ocular characteristic measurement unit 40, and then the process proceeds to Step S8. In Step S7, as described above, the ocular characteristic of the subject eye ER is measured by the ocular characteristic measurement unit 40. In Step S7, the cornea shape and the refractive power (optical characteristic) of the subject eye ER are measured by the ocular characteristic measurement unit 40.

In Step S8, following the measurement of the ocular characteristic of the subject eye ER by the ocular characteristic measurement unit 40 (first measurement unit) in Step S7, the switching control from the ocular characteristic measurement mode to the intraocular pressure measurement mode is conducted, and then the process proceeds to Step S9. In Step S8, as described above, the ocular characteristic measurement mode is switched to the intraocular pressure measurement mode by appropriately driving the Y-axis driver 12a, the Z-axis driver 12b, and the X-axis driver 12c of the driver 12. In this example, the ocular characteristic measurement mode in which the subject eye ER is positioned on the main optical axis O10 of the ocular characteristic measurement unit 40 (refer to FIGs. 5(b) and FIG. 7(b)) is switched to the intraocular pressure measurement mode in which the subject eye ER is positioned on the optical axis O1 of the intraocular pressure measurement unit 20 (refer to FIGs. 5(a) and FIG. 10(a)).

In Step S9, following the switching control from the ocular characteristic measurement mode to the intraocular pressure measurement mode in Step S8, the automatic alignment of the intraocular pressure measurement unit 20 (second measurement unit) relative to the subject eye ER is executed, and then the process proceeds to Step S10. In Step S9, in the intraocular pressure measurement unit 20, the automatic alignment is executed by appropriately moving the apparatus main body 13 to the base 11 based on the position of the subject eye ER in the X and Y directions, which is obtained from the anterior eye observation optical system 21, and the calculation results output from the Z-alignment detection corrector 32. Then, the apparatus main body 13 is moved on the positive side in the Z-axis direction, and the intraocular pressure measurement unit 20 comes close to the subject eye ER, and the leading end of the airflow blowing nozzle 21b is thereby positioned at the second set operating distance d2 from the subject eye ER. The subject eye ER is thus positioned on the optical axis O1 of the intraocular pressure measurement unit 20, the leading end of the intraocular pressure measurement unit 20 is positioned at the second set operating distance d2 from the subject eye ER, and the intraocular pressure measurement unit 20 is appropriately positioned relative to the subject eye ER.

In Step S10, following the executing of the automatic alignment of the intraocular pressure measurement unit 20 (second measurement unit) relative to the subject eye ER in Step S9, the intraocular pressure of the subject eye ER is measured by the intraocular pressure measurement unit 20 (second measurement unit), and then the process proceeds to Step S11. As described above, in Step S10, the intraocular pressure of the subject eye ER is measured by the intraocular pressure measurement unit 20.

In Step S11, following the measurement of the intraocular pressure of the subject eye ER with the intraocular pressure measurement unit 20 (second measurement unit) in Step S10, the automatic alignment of the intraocular pressure measurement unit 20 (second measurement unit) relative to the subject eye ER is executed, and then the process proceeds to Step S12. In Step S11, similar to Step S9, the automatic alignment relative to the subject eye ER is executed. The subject eye ER is positioned on the optical axis O1 of the intraocular pressure measurement unit 20, the leading end of the intraocular pressure measurement unit 20 is positioned at the second set operating distance d2 from the subject eye ER, and the intraocular pressure measurement unit 20 is thereby appropriately positioned relative to the subject eye ER. In Step S11, upon the completion of the automatic alignment, the position (coordinate position) of the automatically aligned apparatus main body 13 (intraocular pressure measurement unit 20) in each of the X, Y, and Z-axis directions is stored. This position to be stored may be only the position in the X-axis direction. In Step S11, the apparatus main body 13 may be moved to that position that allows to detect the position of the bright point image (cornea apex Ea) or the center position of the pupil Ep in the image (data) of the anterior eye (cornea Ec) obtained by the anterior eye observation optical system 21 (second observation optical system) of the intraocular pressure measurement unit 20 (second measurement unit). This movement in Step S11 is to determine the standard position for moving the apparatus main body 13 (intraocular pressure measurement unit 20) by using the distance between both eyes dm in Step S12. In this case, as long as the image (data) includes the center position of the pupil Ep of the subject eye E or the position of the bright point image (cornea apex Ea) formed by the XY alignment index projection optical system 22, the controller 33 recognizes the gap (size and direction) between those positions and the present position of the optical axis O1 of the intraocular pressure measurement unit 20. The controller 33 therefore determines the standard position in Step S12 by using the gap as the correction value.

In Step S12, following the execution of the automatic alignment of the intraocular pressure measurement unit 20 relative to the subject eye ER in Step S11, the right and left switching movement of the intraocular pressure measurement unit 20 (second measurement unit) relative to both subject eyes E is conducted by using the distance between both eyes dm, and then the process proceeds to Step S13. In Step S12, as the intraocular pressure of the subject eye EL has not been measured yet although the intraocular pressure of the subject eye ER has been measured, the rough alignment relative to the subject eye ER is executed by moving the apparatus main body 13 to the subject eye EL (positive side in X-axis direction). More specifically, in Step S12, at first, the airflow blowing nozzle 21b is moved away from the subject eye ER by moving back the apparatus main body 13 (moving on negative side in Z-axis direction). In this embodiment, the apparatus main body 13 is moved back to the back end in the Z-axis direction movable range. After that, in Step S12, the apparatus main body 13 is moved to the subject eye EL (basically positive side in X-axis direction, and in some cases, movement in Y-axis direction and Z-axis direction) at the stored binocular distance between both eyes dm calculated in Step S6. The right and left switching movement of the subject eye E (in this embodiment, from subject eye ER to subject eye EL) is therefore completed. Namely, in Step S12, the apparatus main body 13, namely, the intraocular pressure measurement unit 20 is moved to the subject eye EL at the distance between both eyes dm with the appropriate position of the intraocular pressure measurement unit 20 relative to the subject eye ER by the automatic alignment in S11 as a standard. In addition, in Step S11, when the apparatus main body 13 is moved to the positon that detects the position of the bright point image (cornea apex Ea) or the center position of the pupil Ep in the image (data) of the anterior eye (cornea Ec) obtained by the anterior eye observation optical system 21 (second observation optical system) of the intraocular pressure measurement unit 20 (second measurement unit), the standard position that moves the apparatus main body 13 at the distance between both eyes dm is determined by using the gap as the correction value as described above.

In Step S13, following the right and left switching movement of the intraocular pressure measurement unit 20 (second measurement unit) relative to both subject eyes E with the distance between both eyes dm in Step S12, the automatic alignment of the intraocular pressure measurement unit 20 (second measurement unit) relative to the subject eye EL is executed, and then the process proceeds to Step S13. In Step S13, similar to Step S9, the automatic alignment relative to the subject eye EL is executed. Then, the apparatus main body 13 is moved on the positive side in the Z-axis direction, the intraocular pressure measurement unit 20 comes close to the subject eye EL, and the leading end of the airflow blowing nozzle 21b is thereby positioned at the second set operating distance from the subject eye EL. The subject eye EL is thus positioned on the optical axis O1 of the intraocular pressure measurement unit 20, the leading end of the intraocular pressure measurement unit 20 is positioned at the second set operating distance d2 from the subject eye EL, and the intraocular pressure measurement unit 20 is appropriately positioned relative to the subject eye EL.

In Step S14, following the automatic alignment of the intraocular pressure measurement unit 20 (second measurement unit) relative to the subject eye EL in Step S13, the intraocular pressure of the subject eye EL is measured by the intraocular pressure measurement unit 20 (second measurement unit), and the right and left switching operation movement process (right and left switching movement method) is completed. In Step S14, as described above, the intraocular pressure of the subject eye EL is measured by the intraocular pressure measurement unit 20.

Next, the operation regarding the right and left eyes switching method in the measurement of the ocular characteristic of both subject eyes E with the ocular characteristic measurement unit 40 (first measurement unit) and the measurement of the intraocular pressure of both subject eyes E with the intraocular pressure measurement unit 20 (second measurement unit) will be described.

At first, when the operation for starting the measurement with the ocular characteristic measurement unit 40 (first measurement unit) is executed, in Step S1 → Step S2 in the flowchart of FIG. 9, the ocular characteristic of the subject eye EL is thereby measured by the ocular characteristic measurement unit 40 (first measurement unit) after executing the automatic alignment relative to the subject eye EL (refer to FIG. 7(a)). After that, in Step S3 → Step S4 → Step S5 → Step S6 in the flowchart of FIG. 9, the distance between both eyes dm of both subject eyes E (subject eye EL and subject eye ER) is calculated based on the ocular characteristic measurement unit 40 (first measurement unit) appropriately positioned relative to the subject eye EL in Step S3 and the ocular characteristic measurement unit 40 (first measurement unit) appropriately positioned relative to the subject eye ER in Step S5. After that, in Step S7 → Step S8 in the flowchart of FIG. 9, the ocular characteristic of the subject eye ER is measured by the ocular characteristic measurement unit 40 (first measurement unit) appropriately positioned relative to the subject eye ER (refer to FIG. 7 (b)), and the measurement mode is switched to the intraocular pressure measurement mode (refer to from FIGs. 5(b) to 5(a)).

In Step S9 → Step S10 in the flowchart of FIG.9, after executing the automatic alignment relative to the subject eye ER, the intraocular pressure of the subject eye ER is measured by the intraocular pressure measurement unit 20 (second measurement unit) (refer to FIG. 10(a)). After that, in Step S11 in the flowchart of FIG. 9, the automatic alignment is executed, and the intraocular pressure measurement unit 20 (second measurement unit) is appropriately positioned relative to the subject eye ER (refer to FIG. 10(a)). In Step S12 in the flowchart of FIG. 9, the intraocular pressure measurement unit 20 (second measurement unit) is moved to the subject eye EL at the distance between both eyes dm with the position suitable to the subject eye ER by the automatic alignment in Step S11 as a standard. After that, in Step S13 → Step S14, after executing the automatic alignment relative to the subject eye EL, the intraocular pressure of the subject eye EL is measured by the intraocular pressure measurement unit 20 (second measurement unit) (refer to FIG. 10(d)).

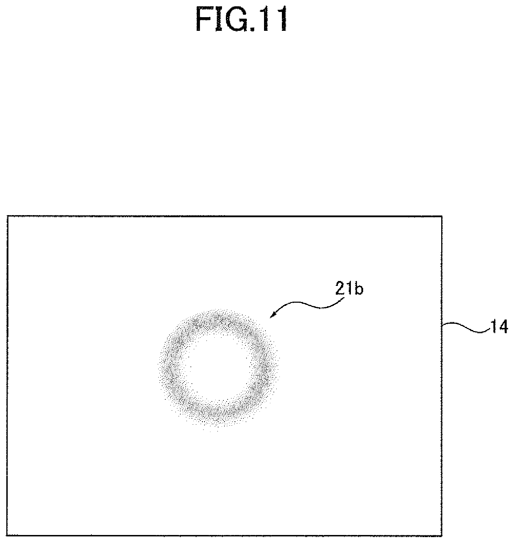

In the ophthalmologic apparatus 10, the right and left subject eyes E are switched for the measurement with the intraocular pressure measurement unit 20 (second measurement unit) by using the distance dm of both subject eyes E obtained for measuring the right and left subject eyes E of the subject with the ocular characteristic measurement unit 40 (first measurement unit). This is because the following problem occurs when the right and left subject eyes E are switched by the detection of the pupil Ep with the intraocular pressure measurement unit 20. Hereinafter, the problem will be described with reference to FIGs. 10 and 11. As this problem also occurs in the ophthalmologic apparatus 10, the description will be given with the ophthalmologic apparatus 10.

In the intraocular pressure measurement unit 20 as the second measurement unit, the second set operating distance d2 is set to a very small value as described above. For this reason, after the intraocular pressure measurement unit 20 measures one subject eye E (for example, subject eye ER) (refer to FIG. 10(a)), the intraocular pressure measurement unit 20 is required to be once moved away from the subject (each subject eye E) at a distance larger than the second set operating distance d2, and then to be moved in the X-axis direction (refer to FIGs. 10(b) and (c)). This is because that if the intraocular pressure measurement unit 20 is moved in the X-axis direction while being set at the second set operating distance d2, the leading end (airflow blowing nozzle 21b) interferes with the subject (mainly nose).

In the intraocular pressure measurement unit 20, the image of the anterior eye is obtained by the CCD camera 21i of the anterior eye observation optical system 21, and the image (data) is output to the controller 33. In the intraocular pressure measurement unit 20, similar to the ocular characteristic measurement unit 40, the position of the pupil Ep of the subject eye E (cornea Ec) can be detected in the input image (data). However, in the intraocular pressure measurement unit 20, the second set operating distance d2 is set to a very small value, and the intraocular pressure measurement unit 20 is moved away from the subject than the second set operating distance d2 when the intraocular pressure measurement unit 20 moves in the X-axis direction as described above. As the anterior eye observation optical system 21 is provided for the measurement with the intraocular pressure measurement unit 20, the anterior eye observation optical system 21 is appropriately focused on the subject eye E set at the second set operating distance d2 of the intraocular pressure measurement unit 20. Consequently, in the intraocular pressure measurement unit 20 (controller 33), the image of the anterior eye (pupil Ep) is unclear, and it becomes difficult to appropriately detect the pupil Ep.

In addition, in the intraocular pressure measurement unit 20 (ophthalmologic apparatus 10), the position of the CCD camera 21i on the optical axis O1 is set in the second focal position f2 (refer to FIG. 3) through the focusing mechanism 21D. The image of the subject eye E (anterior eye (cornea Ec)) can be thereby appropriately displayed on the display 14. In the ophthalmologic apparatus 10 (intraocular pressure measurement unit 20), a problem caused by the out-of-focusing is basically solved.

In the intraocular pressure measurement unit 20, the light flux passes outside the airflow blowing nozzle 21b, transmits through the anterior eye window glass 21c (including the later-described glass plate 34b), the chamber window glass 21d, the half mirror 21g, and the half mirror 21e, and is condensed by the objective lens 21f. The anterior eye observation optical system 21 obtains the anterior eye image (light flux) of the subject eye E (refer to FIG. 3) by forming the image on the CCD camera 21i (light-receiving surface). For this reason, the image (on screen of display 14) obtained by the anterior eye observation optical system 21 includes blurred shadow of the unfocused airflow blowing nozzle 21b, as shown in FIG. 11. The unfocused airflow blowing nozzle 21b (shadow) may be misdetected as the pupil Ep. This is because that when the pupil Ep is detected in an unfocused state, the pupil Ep and the airflow blowing nozzle 21b (shadow) show similar images in the image (on screen of display 14).

The misdetection of the pupil Ep may increase a time required for the automatic rough alignment or may cause the failure of the rough alignment. In the intraocular pressure measurement unit 20 (second measurement unit) in which the second set operating distance d2 is set to a very small value, it is preferable to improve the accuracy of the automatic rough alignment when automatically moving the intraocular pressure measurement unit 20 from the position suitable to one subject eye E (for example, subject eye E) to the position suitable to the other subject eye E.

On the other hand, the ophthalmologic apparatus 10 as one example of the ophthalmologic apparatus according to the present invention obtains the distance between both eyes dm of both subject eyes E when measuring the right and left subject eyes E of the subject with the ocular characteristic measurement unit 40 (first measurement unit), and the right and left subject eyes are switched by using the obtained distance between both eyes dm in the measurement with the intraocular pressure measurement unit 20 (second measurement unit). For this reason, in the ophthalmologic apparatus 10, the intraocular pressure measurement unit 20 (second measurement unit) is moved to the other subject eye E by using the distance between both eyes dm without using the second observation optical system (anterior eye observation optical system 21) of the intraocular pressure measurement unit 20 (second measurement unit) which may misdetect the pupil Ep. The intraocular pressure measurement unit 20 is thereby reliably moved to the position facing the other subject eye side E. In the ophthalmologic apparatus 10, the intraocular pressure measurement unit 20 (second measurement unit) is automatically and appropriately moved to the position suitable to the other subject eye E.

In the ophthalmologic apparatus 10, the pupil Ep detected by the anterior eye observation optical system 21 of the intraocular pressure measurement unit 20 (second measurement unit) is not used for switching the right and left subject eyes E in the measurement with the intraocular pressure measurement unit 20 (second measurement unit). The ophthalmologic apparatus 10 is prevented from being affected by the misdetection of the pupil Ep by the anterior eye observation optical system 21.

In the ophthalmologic apparatus 10, the intraocular pressure measurement unit 20 (second measurement unit) can be reliably moved to the position facing the other subject eye E (pupil Ep). The intraocular pressure measurement unit 20 (second measurement unit) can be appropriately and smoothly aligned to the other subject E. In the ophthalmologic apparatus 10, the intraocular pressure measurement unit 20 (second measurement unit) can be therefore automatically and appropriately moved to the positon suitable to the other subject eye E.

In the ophthalmologic apparatus 10, the pupil Ep detected by the anterior eye observation optical system 21 of the intraocular pressure measurement unit 20 (second measurement unit) is not used. The intraocular pressure measurement unit 20 (second measurement unit) can be therefore automatically and appropriately moved to the position suitable to the other subject eye E without setting the position of the CCD camera 21i on the optical axis O1 to the second focal position f2 through the focusing mechanism 21D provided in the intraocular pressure measurement unit 20 (ophthalmologic apparatus 10). Accordingly, in the ophthalmologic apparatus 10, the focusing mechanism 21D of the intraocular pressure measurement unit 20 (ophthalmologic apparatus 10) is not required. The intraocular pressure measurement unit 20 (second measurement unit) can be therefore automatically and appropriately moved to the position suitable to the other subject E even in an ophthalmologic apparatus which does not include a mechanism corresponding to the focusing mechanism 21D.

In the ophthalmologic apparatus 10, after one subject eye E is measured by the intraocular pressure measurement unit 20 (second measurement unit), the intraocular pressure measurement unit 20 (second measurement unit) is aligned to one subject eye E, and then the intraocular pressure measurement unit 20 (second measurement unit) is moved to the other subject eye E at the obtained distance between both eyes dm. Even if the subject moves the face after one subject eye E is measured by the intraocular pressure measurement unit 20, the intraocular pressure measurement unit 20 (second measurement unit) is moved at the distance between both eyes dm with the position of one subject eye E in the moved face as a standard. The intraocular pressure measurement unit 20 (second measurement unit) can be automatically and appropriately moved to the position facing the other subject eye E.

In the ophthalmologic apparatus 10, as the intraocular pressure measurement unit 20 is used as the second measurement unit, after one subject eye E is measured by the intraocular pressure measurement unit 20 (second measurement unit), the intraocular pressure measurement unit 20 (second measurement unit) can be further effectively aligned to the one subject eye E. This is because that, in the intraocular pressure measurement unit 20, as the intraocular pressure is measured by blowing fluid to the cornea Ec of the subject eye E to deform the cornea Ec, some of the subjects may move their face due to the uncomfortable feeling caused by the blowing of the fluid.

In the ophthalmologic apparatus 10, the distance between both eyes dm is obtained based on the position of the ocular characteristic measurement unit 40 (first measurement unit) aligned to one subject eye E and the positon of the ocular characteristic measurement unit 40 (first measurement unit) aligned to the other subject eye E. In the ophthalmologic apparatus 10, the distance between both eyes dm can be therefore further appropriately obtained.

In the ophthalmologic apparatus 10, the distance between both eyes dm is obtained based on the position of the ocular characteristic measurement unit 40 (first measurement unit) aligned to one subject eye E after one subject eye E is measured by the ocular characteristic measurement unit 40 (first measurement unit) and the position of the ocular characteristic measurement unit 40 (first measurement unit) aligned to the other subject eye E after that. In the ophthalmologic apparatus 10, even if the subject moves the face after the one subject eye E is measured by the ocular characteristic measurement unit 40 (first measurement unit), the distance between both eyes dm can be obtained with the position of the one subject eye E of the moved face as a standard. The distance between both eyes dm can be further appropriately obtained.

In the ophthalmologic apparatus 10, after one subject eye E is measured by the ocular characteristic measurement unit 40 (first measurement unit), the ocular characteristic measurement unit 40 (first measurement unit) is moved to the other subject E with the position of the pupil Ep detected based on the image of the anterior eye obtained by the observation optical system 42 of the ocular characteristic measurement unit 40 (first measurement unit) as a target. In this case, as the first set operating distance d1 is set to a relatively large value in the ocular characteristic measurement unit 40 (first measurement unit), the ocular characteristic measurement unit 40 (first measurement unit) can be moved to the other subject eye E without being moved back for switching the right and left subject eyes E. The pupil Ep can be appropriately detected. In the ophthalmologic apparatus 10, the distance between both eyes dm can be obtained with the ocular characteristic measurement unit 40 (first measurement unit) with high accuracy, and the intraocular pressure measurement unit 20 can be moved to the other subject eye E by using the distance between both eyes dm. The intraocular pressure measurement unit 20 (second measurement unit) can be automatically and appropriately moved to the position facing the other subject eye E.

In the ophthalmologic apparatus 10, the controller 33 stores the position of the ocular characteristic measurement unit 40 (first measurement unit) in each of the X, Y, and Z directions in the measurement of one subject eye E and the position of the ocular characteristic measurement unit 40 (first measurement unit) in each of the X, Y, and Z directions in the measurement of the other subject eye E. In the ophthalmologic apparatus 10, the controller 33 obtains the distance between both eyes dm as the distance between right and left subject eyes E by obtaining the difference in both of the positions in each of the X, Y, and Z directions, and stores the obtained distance between both eyes dm. In the ophthalmologic apparatus 10, the intraocular pressure measurement unit 20 (second measurement unit) can be therefore moved by using the displacement (control amount for movement (pulse number in this embodiment)) of each of the Y-axis driver 12a, the Z-axis driver 12b, and the X-axis driver 12c of the driver 12. In the ophthalmologic apparatus 10, the intraocular pressure measurement unit 20 (second measurement unit) can be moved by the same volume and direction (binocular distance dm) as those which move the ocular characteristic measurement unit 40 (first measurement unit) by the driver 12. In the ophthalmologic apparatus 10, the intraocular pressure measurement unit 20 (second measurement unit) can be automatically and appropriately moved to the position facing the other subject eye E while simplifying the control for the movement.

In the ophthalmologic apparatus 10, the distance between both eyes dm is obtained with the cornea apexes Ea (position) of the corneas Ec of both subject eyes E. In the ophthalmologic apparatus 10, the intraocular pressure measurement unit 20 (second measurement unit) that measures the intraocular pressure of the subject eye E with the optical axis O1 being aligned with the cornea apex Ea of the cornea Ec of the subject eye E can be automatically and appropriately moved to the position facing the other subject eye E.

In the ophthalmologic apparatus 10, as the distance between both eyes dm is obtained based on the position of the ocular characteristic measurement unit 40 (first measurement unit) aligned to one subject eye E and the position of the ocular characteristic measurement unit 40 (first measurement unit) aligned to the other subject eye E, the distance between both eyes dm is obtained based on the cornea apexes Ea of both subject eyes E by using the position of the apparatus main body 13. In the ophthalmologic apparatus 10, the intraocular pressure measurement unit 20 (second measurement unit) can be automatically and appropriately moved to the position facing the other subject eye E.

In the ophthalmologic apparatus 10, the distance between both eyes dm can be obtained with the image (data) of the anterior eyes (cornea Ec) obtained by the observation optical system 42 (first observation optical system) of the ocular characteristic measurement unit 40 (first measurement unit). In the ophthalmologic apparatus 10, the distance between both eyes dm can be therefore appropriately obtained without accurately aligning the ocular characteristic measurement unit 40 (first measurement unit) relative to the subject eye E. In the ophthalmologic apparatus 10, the intraocular pressure measurement unit 20 (second measurement unit) can be thus automatically and appropriately moved to the position facing the other subject eye E.

In the ophthalmologic apparatus 10, the distance between both eyes dm can be obtained with the center positions of the pupils Ep in the image (data) of the anterior eyes (cornea Ec) obtained by the observation optical system 42 (first observation optical system) of the ocular characteristic measurement unit 40 (first measurement unit). In the ophthalmologic apparatus 10, the intraocular pressure measurement unit 20 (second measurement unit) can be therefore automatically and appropriately moved to the position facing the other subject eye E without the optical axis being aligned with the cornea apex Ea. This is because the optical axis may be aligned with the center position of the pupil Ep when the refractive power is measured by the ocular characteristic measurement unit 40 (first measurement unit), and the alignment is may be executed around opacity when the subject eye E is cataract having opacity in the lens. In this case, it is preferable to detect the gap between the cornea apex Ea and the central position of the pupil Ep of the subject eye E in the image (data) of the anterior eye (cornea Ec) obtained by the observation optical system 42 (first observation optical system) of the ocular characteristic measurement unit 40 (first measurement unit), and correct the gap after moving the intraocular pressure measurement unit 20 (second measurement unit) with the distance between both eyes dm.

In the ophthalmologic apparatus 10, the magnification of the anterior eye observation optical system 21 (second observation optical system) in the intraocular pressure measurement unit 20 (second measurement unit) can be set higher than that of the observation optical system 42 (first observation optical system) in the ocular characteristic measurement unit 40 (second measurement unit). In the ophthalmologic apparatus 10, as the alignment accuracy of the intraocular pressure measurement unit 20 (second measurement unit) can be improved, the measurement result of the intraocular pressure of the subject eye E by the intraocular pressure measurement unit 20 (second measurement unit) can be prevented from being affected by the lowered alignment accuracy. Namely, in the ophthalmologic apparatus 10, the appropriate measurement result of the intraocular pressure of the subject eye E can be reliably obtained by the intraocular pressure measurement unit 20 (second measurement unit). As the above setting narrows the imaging range of the anterior eye observation optical system 21 (second observation optical system) of the intraocular pressure measurement unit 20 (second measurement unit), it becomes difficult to detect the pupil Ep, and as the above setting reduces the focal depth, an out-of-focus image is obtained. For this reason, in the ophthalmologic apparatus 10, it becomes further difficult to detect the pupil Ep by the anterior eye observation optical system 21 (second observation optical system) of the intraocular pressure measurement unit 20 (second measurement unit). In the ophthalmologic apparatus 10, even when the magnification is set as described above, the intraocular pressure measurement unit 20 (second measurement unit) can be effectively moved to the other subject eye E by using the distance between both eyes dm without using the anterior eye observation optical system 21 (second observation optical system) of the intraocular pressure measurement unit 20 (second measurement unit).

In the ophthalmologic apparatus 10 as one embodiment of the ophthalmologic apparatus according to the present invention, the intraocular pressure measurement unit 20 (second measurement unit) set at the second set operating distance d2 having a value smaller than that of the first set operating distance d1 is automatically and appropriately moved from the position suitable to one subject eye E to the position suitable to the other subject eye E.

In the ophthalmologic apparatus of the present invention, the second measurement unit is set to the second set operating distance having a very small value. For this reason, when the second measurement unit is moved from the position suitable to one subject eye to the position suitable to the other subject eye, the second measurement unit is moved back to be away from the subject eye for preventing the interference with the nose of the subject, for example. The second measurement unit thus detects the subject eye (pupil) at a distance significantly longer than the second set operating distance. Therefore, the observation optical system provided in the second measurement unit to be set at the second set operating distance may not appropriately detect the subject eye (pupil). To solve such a problem, the ophthalmologic apparatus of the present invention includes a first measurement unit set at a first set operating distance to measure a subject eye of a subject, a second measurement unit set at a second set operating distance shorter than the first set operating distance to measure the subject eye, a driver that moves the first measurement unit and the second measurement unit relative to the subject eye, and a controller that controls the first measurement unit, the second measurement unit, and the driver, wherein the controller obtains a distance between both eyes of right and left subject eyes when the first measurement unit is moved to measure the right and left subject eyes of the subject, and after one subject eye is measured with the second measurement unit, the controller moves the second measurement unit to the other subject eye at the obtained distance between both eyes. With this configuration, the second measurement unit set at the second set operating distance having a value smaller than that of the first set operating distance can be automatically and appropriately moved from the position suitable to one subject eye to the position suitable to the other subject eye.

In addition to the above configuration, the controller executes alignment of the second measurement unit relative to the one subject eye after the one subject eye is measured with the second measurement unit, and then moves the second measurement unit to the other subject eye at the obtained distance between both eyes. With this configuration, the second measurement unit can be automatically and further appropriately moved to the positon facing the other subject eye.

In addition to the above configuration, the controller obtains the distance between both eyes based on a position of the first measurement unit aligned to the one subject eye and a position of the first measurement unit aligned to the other subject eye. With this configuration, the distance between both eyes can be further appropriately obtained.

In addition to the above configuration, the controller obtains the distance between both eyes based on the position of the first measurement unit aligned to the one subject eye after the one subject eye is measured with the first measurement unit and the position of the first measurement unit aligned to the other subject eye after the alignment to the one subject eye. With this configuration, the distance between both eyes can be appropriately obtained even when the subject moves the face.

In addition to the above configuration, the second measurement unit is an intraocular pressure measurement unit that blows fluid to a cornea of the subject eye to measure an intraocular pressure by deforming the cornea. With this configuration, the second measurement unit can be automatically and further appropriately moved to the position facing the other subject eye even when the subject moves the face due to the blowing of the fluid.

In addition to the above configuration, the first measurement unit includes a first observation optical system that obtains an image of an anterior eye of the subject eye, and the controller obtains a position of a pupil in the anterior eye based on the image of the anterior eye obtained by the first observation optical system, and moves the first measurement unit to the other subject eye with the position of the pupil of the other subject eye obtained based on the image of the anterior eye as a target after the one subject eye is measured with the first measurement unit. With this configuration, the second measurement unit can be automatically and appropriately moved to the position facing the other subject eye.

In addition to the above configuration, the controller obtains, as the distance between both eyes, displacement of the first measurement unit in each of an up and down direction, a right and left direction, and a back and forth direction, which are orthogonal to each other, in the ophthalmologic apparatus, and moves the second measurement unit by the displacement in each of the up and down direction, the right and left direction, and the back and forth direction obtained as the distance between both eyes. With this configuration, the second measurement unit can be automatically and further appropriately moved to the position facing the other subject eye while simplifying the control for the movement.

In addition to the above configuration, the controller obtains the distance between both eyes based on positions of cornea apexes of the right and left subject eyes. With this configuration, when the second measurement unit executes the measurement with the optical axis being aligned with the cornea apex (position) of the cornea of the subject, the second measurement unit can be automatically and further appropriately moved to the position facing the other subject eye,

In addition to the above configuration, the first measurement unit includes a first observation optical system that obtains an image of an anterior eye of the subject eye, the second measurement unit includes a second observation optical system that obtains an image of an anterior eye of the subject eye, and the second observation system has a magnification higher than that of the first observation optical system. With this configuration, the second measurement unit can be further effectively moved to the other subject eye with the distance between both eyes.

In the above embodiment, the ophthalmologic apparatus 10 as one embodiment of the ophthalmologic apparatus according to the present invention is described. However, the ophthalmologic apparatus according to the present invention is not limited to the above embodiment as long as it includes a first measurement unit set at a first set operating distance to measure a subject eye of a subject, a second measurement unit set at a second set operating distance shorter than the first set operating distance to measure the subject eye, a driver that moves the first measurement unit and the second measurement unit relative to the subject eye; and a controller that controls the first measurement unit, the second measurement unit, and the driver, wherein the controller obtains a distance between both eyes of right and left subject eyes when the first measurement unit is moved to measure the right and left subject eyes of the subject, and after one subject eye is measured with the second measurement unit, the controller moves the second measurement unit to the other subject eye at the obtained distance between both eyes.

In the above embodiment, the alignment of the intraocular pressure measurement unit 20 (second measurement unit) relative to one subject eye E is executed after one subject eye E is measured with the intraocular pressure measurement unit 20 (second measurement unit), and then the intraocular pressure measurement unit 20 (second measurement unit) is moved to the other subject eye E at the obtained distance between both eyes dm. However, it is not limited to the above embodiment. The alignment of the intraocular pressure measurement unit 20 (second measurement unit) relative to one subject eye E is completed when one subject eye E is measured with the intraocular pressure measurement unit 20 (second measurement unit). Therefore, after one subject eye E is measured with the intraocular pressure measurement unit 20 (second measurement unit), the intraocular pressure measurement unit 20 (second measurement unit) may be moved to the other subject E at the obtained distance between both eyes from that position.

In the above embodiment, the distance between both eyes dm is obtained based on a position of the ocular characteristic measurement unit 40 (first measurement unit) aligned to one subject eye E after one subject eye is measured with the ocular characteristic measurement unit 40 (the first measurement unit) and a position of the ocular characteristic measurement unit 40 (first measurement unit) aligned to the other subject eye E after the alignment to one subject eye. However, it is not limited to the above embodiment. The alignment of the ocular characteristic measurement unit 40 (first measurement unit) relative to one subject eye E is completed when one subject eye E is measured with the ocular characteristic measurement unit 40 (first measurement unit). Therefore, the distance between both eyes dm may be obtained based on the position where one subject eye E is measured with the ocular characteristic measurement unit 40 (first measurement unit) and the position of the ocular characteristic measurement unit 40 (first measurement unit) aligned to the other subject eye E after that.

In the above embodiment, the intraocular pressure measurement unit 20 is installed as the second measurement unit. However, it is not limited to the above embodiment. An intraocular pressure measurement unit having a different optical configuration, different arrangement of each optical member, and a different measurement principle, or a pachymeter that measures a thickness of a cornea may be used as long as the second set operating distance d2 is set to a value smaller than that of the first set operating distance d1.

In the above embodiment, the ocular characteristic measurement unit 40 (first measurement unit) is moved to the other subject E with the position of the pupil Ep detected based on the image of the anterior eye obtained by the observation optical system 42 as a target. However, it is not limited to the above embodiment as long as the ocular characteristic measurement unit 40 (first measurement unit) is automatically moved to the position facing the other subject eye E after one subject eye E is measured with the ocular characteristic measurement unit 40 (first measurement unit).

In the above embodiment, the ocular characteristic measurement unit 40 is installed as the first measurement unit. However, it is not limited to the above embodiment. An ocular characteristic measurement unit having a different optical configuration, different arrangement of each optical member, a different measurement principle, and a different measurement type may be used as long as it receives the reflection light from the subject eye E and measures the optical characteristic of the subject eye E.

In the above embodiment, the position of the CCD camera 21i is switched between the first focal position f1 and the second focal position f2 in the intraocular pressure measurement unit 20. However, it is not limited to the above embodiment. The movement of the CCD camera 21i can be appropriately set as long as it can be focused on the anterior eye (cornea Ec) of the subject eye E by the movement.

In the above embodiment, the ocular characteristic measurement unit 40 as the first measurement unit is integrally provided below the intraocular pressure measurement unit 20 as the second measurement unit. However, it is not limited to the above embodiment. The positional relationship between the first and second measurement units may be appropriately set, and the first measurement unit and the second measurement unit may be independently moved.

Although the method for operating an ophthalmologic apparatus of the present invention has been described in terms of the embodiment, it is not limited thereto. It should be appreciated that variations or modifications may be made in the embodiment described without departing from the scope of the present invention.

## Claims

1. A method for operating an ophthalmologic apparatus comprising:
a first measurement unit (40) set at a first set operating distance (d1) to measure a subject eye (E) of a subject;
a second measurement unit (20) set at a second set operating distance (d2) shorter than the first set operating distance (d1) to measure the subject eye (E);
a driver (12) that moves the first measurement unit (40) and the second measurement unit (20) relative to the subject eye (E); and
a controller (33) that controls the first measurement unit (40), the second measurement unit (20), and the driver (12), **characterized in that** the method comprises the steps of
obtaining by the controller (33) a displacement of the first measurement unit (40) in each of an up and down direction, a right and left direction, and a back and forth direction, which are orthogonal to each other, in the ophthalmologic apparatus as a distance between both eyes (dm) of right and left subject eyes (E) when the first measurement unit (40) is **characterized by** the moved to measure the right and left subject eyes (E) of the subject, and step of
moving by the controller (33), after one subject eye (E) is measured with the second measurement unit (20), the second measurement unit (20) to the other subject eye (E) by the displacement in each of the up and down direction, the right and left direction, and the back and forth direction obtained as the distance between both eyes (dm).

2. The method for operating an ophthalmologic apparatus according to claim 1, wherein the controller (33) executes alignment of the second measurement unit (20) relative to the one subject eye (E) after the one subject eye (E) is measured with the second measurement unit (20), and then moves the second measurement unit (20) to the other subject eye (E) at the obtained distance between both eyes (dm).

3. The method for operating an ophthalmologic apparatus according to claim 1 or 2, wherein the controller (33) obtains the distance between both eyes (dm) based on a position of the first measurement unit (40) aligned to the one subject eye (E) and a position of the first measurement unit (40) aligned to the other subject eye (E).

4. The method for operating an ophthalmologic apparatus according to claim 3, wherein the controller (33) obtains the distance between both eyes (dm) based on the position of the first measurement unit (40) aligned to the one subject eye (E) after the one subject eye (E) is measured with the first measurement unit (40) and the position of the first measurement unit (40) aligned to the other subject eye (E) after the alignment to the one subject eye (E).

5. The method for operating an ophthalmologic apparatus according to any one of claims 1 to 4, wherein the second measurement unit (20) is an intraocular pressure measurement unit (20) that blows fluid to a cornea of the subject eye (E) to measure an intraocular pressure by deforming the cornea.

6. The method for operating an ophthalmologic apparatus according to any one of claims 1 to 5, wherein
the first measurement unit (40) includes a first observation optical system (42) that obtains an image of an anterior eye of the subject eye (E), and
the controller (33) obtains a position of a pupil in the anterior eye based on the image of the anterior eye obtained by the first observation optical system (42), and moves the first measurement unit (40) to the other subject eye (E) with the position of the pupil of the other subject eye (E) obtained based on the image of the anterior eye as a target after the one subject eye (E) is measured with the first measurement unit (40).

7. The method for operating an ophthalmologic apparatus according to any one of claims 1 to 6, wherein the controller (33) obtains the distance between both eyes (dm) based on positions of cornea apexes of the right and left subject eyes.

8. The method for operating an ophthalmologic apparatus according to any one of claims 1 to 7, wherein
the first measurement unit (40) includes a first observation optical system (44) that obtains an image of an anterior eye of the subject eye (E),
the second measurement unit (20) includes a second observation optical system (21) that obtains an image of an anterior eye of the subject eye (E), and
the second observation optical system (21) has a magnification higher than that of the first observation optical system (44).

## Patentansprüche

1. Verfahren zum Betreiben einer ophthalmologischen Vorrichtung, die Folgendes umfasst:
eine erste Messeinheit (40), die auf einen ersten eingestellten Betriebsabstand (d1) eingestellt wird, um ein Probandenauge (E) eines Probanden zu messen;
eine zweite Messeinheit (20), die auf einen zweiten eingestellten Betriebsabstand (d2) eingestellt wird, der kürzer als der erste eingestellte Betriebsabstand (d1) ist, um das Probandenauge (E) zu messen;
eine Antriebseinheit (12), die die erste Messeinheit (40) und die zweite Messeinheit (20) in Bezug auf das Probandenauge (E) bewegt; und
eine Steuerung (33), die die erste Messeinheit (40), die zweite Messeinheit (20) und die Antriebseinheit (12) steuert, **dadurch gekennzeichnet, dass** das Verfahren die folgenden Schritte umfasst:
Erhalten einer Verlagerung der ersten Messeinheit (40) in einer Aufwärts-Abwärts-Richtung, einer Rechts-Links-Richtung und einer Rückwärts-Vorwärts-Richtung, die zueinander senkrecht stehen, durch die Steuerung (33) in der ophthalmologischen Vorrichtung als einen Abstand zwischen beiden Augen (dm) eines rechten und eines linken Probandenauges (E), wenn die erste Messeinheit (40) bewegt wird, um das rechte und das linke Probandenauge (E) des Probanden zu messen, und **gekennzeichnet durch** den folgenden Schritt:
Bewegen der zweiten Messeinheit (20), nachdem ein Probandenauge (E) mit der zweiten Messeinheit (20) gemessen wurde, durch die Steuerung (33) zu dem anderen Probandenauge (E) durch die Verlagerung in der Aufwärts-AbwärtsRichtung, der Rechts-Links-Richtung und der Rückwärts-Vorwärts-Richtung, die als der Abstand zwischen beiden Augen (dm) erhalten wurde.

2. Verfahren zum Betreiben einer ophthalmologischen Vorrichtung nach Anspruch 1, wobei die Steuerung (33) eine Justierung der zweiten Messeinheit (20) in Bezug auf das eine Probandenauge (E) ausführt, nachdem das eine Probandenauge (E) mit der zweiten Messeinheit (20) gemessen wurde, und dann die zweite Messeinheit (20) zu dem anderen Probandenauge (E) bei dem erhaltenen Abstand zwischen beiden Augen (dm) bewegt.

3. Verfahren zum Betreiben einer ophthalmologischen Vorrichtung nach Anspruch 1 oder 2, wobei die Steuerung (33) den Abstand zwischen beiden Augen (dm) auf der Basis einer Position der ersten Messeinheit (40), die auf das eine Probandenauge (E) justiert ist, und einer Position der ersten Messeinheit (40), die auf das andere Probandenauge (E) justiert ist, erhält.

4. Verfahren zum Betreiben einer ophthalmologischen Vorrichtung nach Anspruch 3, wobei die Steuerung (33) den Abstand zwischen beiden Augen (dm) auf der Basis der Position der ersten Messeinheit (40), die auf das eine Probandenauge (E) justiert ist, nachdem das eine Probandenauge (E) mit der ersten Messeinheit (40) gemessen wurde, und der Position der ersten Messeinheit (40), die nach dem Justieren auf das eine Probandenauge (E) auf das andere Probandenauge (E) justiert wird, erhält.

5. Verfahren zum Betreiben einer ophthalmologischen Vorrichtung nach einem der Ansprüche 1 bis 4, wobei die zweite Messeinheit (20) eine Innenaugendruck-Messeinheit (20) ist, die Fluid auf die Hornhaut des Probandenauges (E) bläst, um einen Innenaugendruck durch Verformen der Hornhaut zu messen.

6. Verfahren zum Betreiben einer ophthalmologischen Vorrichtung nach einem der Ansprüche 1 bis 5, wobei
die erste Messeinheit (40) ein erstes optisches Beobachtungssystem (42) umfasst, das ein Bild eines Vorderauges des Probandenauges (E) erhält, und
die Steuerung (33) eine Position einer Pupille des Vorderauges auf der Basis des Bilds des Vorderauges, das durch das erste optische Beobachtungssystem (42) erhalten wurde, erhält, und die erste Messeinheit (40) zu dem anderen Probandenauge (E) bewegt, wobei die Position der Pupille des anderen Probandenauges (E) auf der Basis des Bilds des Vorderauges als ein Ziel erhalten wird, nachdem das eine Probandenauge (E) mit der ersten Messeinheit (40) gemessen wurde.

7. Verfahren zum Betreiben einer ophthalmologischen Vorrichtung nach einem der Ansprüche 1 bis 6, wobei die Steuerung (33) den Abstand zwischen beiden Augen (dm) auf der Basis von Positionen der Hornhautscheitelpunkte des rechten und des linken Probandenauges erhält.

8. Verfahren zum Betreiben einer ophthalmologischen Vorrichtung nach einem der Ansprüche 1 bis 7, wobei
die erste Messeinheit (40) ein erstes optisches Beobachtungssystem (44) umfasst, das ein Bild eines Vorderauges des Probandenauges (E) erhält,
die zweite Messeinheit (20) ein zweites optisches Beobachtungssystem (21) umfasst, das ein Bild eines Vorderauges des Probandenauges (E) erhält, und
das zweite optische Beobachtungssystem (21) eine Vergrößerung aufweist, die höher als die des ersten optischen Beobachtungssystems (44) ist.

## Revendications

1. Une méthode de fonctionnement d'un appareil ophtalmologique, comprenant :
une première unité de mesure (40) réglée à une première distance de fonctionnement déterminée (d1) pour mesurer un œil de sujet (E) d'un sujet ;
une seconde unité de mesure (20) réglée à une seconde distance de fonctionnement définie (d2) plus courte que la première distance de fonctionnement définie (d1) pour mesurer l'œil de sujet (E) ;
un entraînement (12) qui déplace la première unité de mesure (40) et la seconde unité de masure (20) par rapport à l'œil de sujet (E) ; et
un contrôleur (33) qui commande la première unité de mesure (40), la seconde unité de mesure (20) et le dispositif d'entraînement (12), **caractérisé en ce que** le procédé comprend les étapes suivantes :
obtention par le contrôleur (33) d'un déplacement de la première unité de mesure (40) dans chacune d'une direction vers le haut et vers le bas, d'une direction vers la droite et vers la gauche et d'une direction vers l'avant et vers l'arrière, qui sont orthogonales les unes par rapport aux autres, dans l'appareil ophtalmologique en tant que distance entre les deux yeux (dm) des yeux droit et gauche de sujet (E) lorsque la première unité de mesure (40) est déplacée pour mesurer les yeux droit et gauche de sujet (E) du sujet, et
**caractérisé par** l'étape suivante
déplacement par le contrôleur (33), après qu'un œil de sujet (E) est mesuré avec la seconde unité de mesure (20), de la seconde unité de mesure (20) vers l'autre œil de sujet (E) par le déplacement dans chacune de la direction vers le haut et vers le bas, de la direction vers la droite et vers la gauche et de la direction vers l'avant et vers l'arrière obtenu en tant que distance entre les deux yeux (dm).

2. La méthode de fonctionnement d'un appareil ophtalmologique selon la revendication 1, dans laquelle le contrôleur (33) exécute l'alignement de la seconde unité de mesure (20) par rapport au premier œil de sujet (E) après que le premier œil de sujet (E) est mesuré avec la seconde unité de mesure (20), et déplace ensuite la seconde unité de mesure (20) vers l'autre œil de sujet (E) à la distance obtenue entre les deux yeux (dm).

3. La méthode de fonctionnement d'un appareil ophtalmologique selon la revendication 1 ou 2, dans laquelle le contrôleur (33) obtient la distance entre les deux yeux (dm) sur la base d'une position de la première unité de mesure (40) alignée sur le premier œil de sujet (E) et d'une position de la première unité de mesure (40) alignée sur l'autre œil de sujet (E).

4. La méthode de fonctionnement d'un appareil ophtalmologique selon la revendication 3, dans laquelle le contrôleur (33) obtient la distance entre les deux yeux (dm) sur la base de la position de la première unité de mesure (40) alignée sur le premier œil de sujet (E) après que le premier œil de sujet (E) est mesuré avec la première unité de mesure (40) et de la position de la première unité de mesure (40) alignée sur l'autre œil de sujet (E) après l'alignement sur le premier œil de sujet (E).

5. La méthode de fonctionnement d'un appareil ophtalmologique selon l'une quelconque des revendications 1 à 4, dans laquelle la seconde unité de mesure (20) est une unité de mesure de pression intraoculaire (20) qui souffle du fluide vers une cornée de l'œil de sujet (E) pour mesurer une pression intraoculaire en déformant la cornée.

6. La méthode de fonctionnement d'un appareil ophtalmologique selon l'une quelconque des revendications 1 à 5, dans laquelle
la première unité de mesure (40) inclut un premier système optique d'observation (42) qui obtient une image d'un œil antérieur de l'œil de sujet (E), et
le contrôleur (33) obtient une position d'une pupille dans l'œil antérieur sur la base de l'image de l'œil antérieur obtenue par le premier système optique d'observation (42), et déplace la première unité de mesure (40) vers l'autre œil de sujet (E) avec la position de la pupille de l'autre œil de sujet (E) obtenue sur la base de l'image de l'œil antérieur en tant que cible une fois que le premier œil de sujet (E) est mesuré avec la première unité de mesure (40).

7. La méthode de fonctionnement d'un appareil ophtalmologique selon l'une quelconque des revendications 1 à 6, dans laquelle le contrôleur (33) obtient la distance entre les deux yeux (dm) sur la base des positions des apex cornéens des yeux droit et gauche de sujet.

8. La méthode de fonctionnement d'un appareil ophtalmologique selon l'une quelconque des revendications 1 à 7, dans laquelle
la première unité de mesure (40) inclut un premier système optique d'observation (44) qui obtient une image d'un œil antérieur de l'œil de sujet (E),
la seconde unité de mesure (20) inclut un second système optique d'observation (21) qui obtient une image d'un œil antérieur de l'œil de sujet (E), et
le second système optique d'observation (21) présente un grossissement supérieur à celui du premier système optique d'observation (44).
